(19) Europäisches Patentamt
European Patent Office
Office européen des brevets

(11) EP 4 361 159 A1

(12) **EUROPEAN PATENT APPLICATION**
published in accordance with Art. 153(4) EPC

(43) Date of publication:
**01.05.2024 Bulletin 2024/18**

(21) Application number: **22828062.4**

(22) Date of filing: **20.04.2022**

(51) International Patent Classification (IPC):
***C07F 5/02*** *(2006.01)* ***A61K 31/69*** *(2006.01)*
***A61K 41/00*** *(2020.01)* ***A61K 51/00*** *(2006.01)*
***A61K 51/04*** *(2006.01)* ***A61P 35/00*** *(2006.01)*

(52) Cooperative Patent Classification (CPC):
**C07F 5/025; A61K 31/69; A61K 41/00; A61K 51/00; A61K 51/04; A61P 35/00; C07F 5/02**

(86) International application number:
**PCT/JP2022/018258**

(87) International publication number:
**WO 2022/270143 (29.12.2022 Gazette 2022/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.06.2021 JP 2021105562**

(71) Applicant: **Stella Pharma Corporation**
**Osaka-shi, Osaka 541-0043 (JP)**

(72) Inventors:
- **MURATA,Yusuke**
**Osaka-shi, Osaka 541-0043 (JP)**
- **TAKENAKA,Hiroshi**
**Osaka-shi, Osaka 541-0043 (JP)**
- **OHTA,Yoichiro**
**Osaka-shi, Osaka 541-0043 (JP)**
- **SUDANI,Aya**
**Osaka-shi, Osaka 541-0043 (JP)**
- **ISHIMURA,Miki**
**Osaka-shi, Osaka 541-0043 (JP)**
- **SUZUKI,Kensuke**
**Osaka-shi, Osaka 541-0043 (JP)**
- **KAI,Hiroyuki**
**Osaka-shi, Osaka 541-0043 (JP)**

(74) Representative: **Cabinet Beau de Loménie**
**158, rue de l'Université**
**75340 Paris Cedex 07 (FR)**

# (54) BORONO-PHENYLALANINE DERIVATIVE

(57) Provided are new compounds that can have the property of being specifically taken up into cancer cells. In the present invention, novel 4-borono-phenylalanine derivatives, borono-phenylalanine derivatives having a heterocyclic skeleton, borono-phenylalanine derivatives having a fused ring structure, or pharmaceutically acceptable salts thereof are prepared. These compounds have the property of being taken up specifically by LAT1 and thus easily taken up specifically by cancer cells in the evaluation of LAT1 and LAT2 selective uptake, and can be conveniently used for neutron capture therapy and the like. A typical example is a compound represented by the following formula (I) or a pharmaceutically acceptable salt thereof.

[Formula 73]

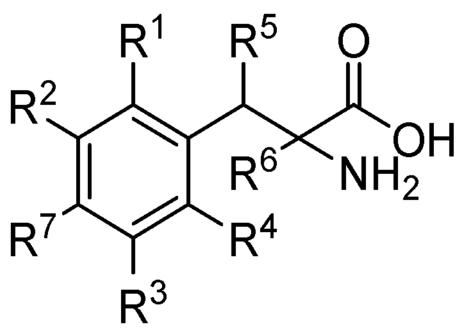

EP 4 361 159 A1

## Description

BACKGROUND OF THE INVENTION

Field of the Invention

The present invention relates to borono-phenylalanine derivatives.

Description of the Related Art

One of the methods for treating cancer is boron neutron capture therapy (BNCT). Boron neutron capture therapy is a therapeutic method in which a boron compound containing a boron-10 isotope ($^{10}B$) is taken up into cancer cells and irradiated with low-energy neutron rays (for example, thermal neutrons) to locally destroy the cancer cells by a nuclear reaction occurring in the cells. In this therapeutic method, since it is important to selectively accumulate a boron compound containing $^{10}B$ in cells of cancer tissue in order to enhance the therapeutic effect, it is necessary to develop a boron compound that is selectively taken up by cancer cells.

As an agent used for BNCT, a 4-borono-phenylalanine derivative in which a boron atom or a boron atom group is introduced into a basic skeleton has been synthesized. As an agent in use in actual clinical practice, there are a 4-borono-phenylalanine derivative (L-BPA) and mercaptoundecahydrododecaborate (BSH). 4-Borono-phenylalanine is taken up by L-type amino acid transporter 1 (LAT1), which is a type of amino acid transporter, as a mimic of phenylalanine. Since expression of LAT1 is enhanced in cancer cells, L-BPA is likely to accumulate and is used for treatment of cancer by utilizing its properties (see, for example, Patent Document 1).

Prior Art Document

Patent Document

Patent Document 1: JP-A-2008-214319

SUMMARY OF THE INVENTION

There is a need for further development of new compounds having the property of being specifically taken up into cancer cells.

An object of the present invention is to provide borono-phenylalanine derivatives.

As a result of intensive studies to solve the above problems, the present inventors have found new derivatives of borono-phenylalanine and have accomplished the present invention.

That is, the present invention provides the following compound.

[1] A compound represented by the following formula (I) or a pharmaceutically acceptable salt thereof:

[Formula 1]

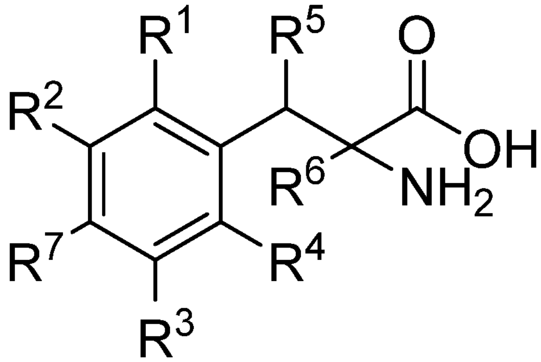

(I)

in the formula (I),

$R^1$, $R^2$, $R^3$, and $R^4$ each independently represent H, halogen, hydroxy, cyano, C1-6 alkyl, C1-6 alkoxy, benzyloxy, C1-6 alkoxy C1-6 alkyl, nitro, C1-6 haloalkyl, aminocarbonyl, C1-C6 alkylaminocarbonyl ($CONR^8R^9$ ($R^8$ and $R^9$ each independently represent H or C1-6 alkyl)), C1-C6 alkoxycarbonyl, C1-C6 alkylcarbonyl, $COOR^{10}$ ($R^{10}$ represents H or C1-6 alkyl, amino, alkylamino ($NR^{11}R^{12}$ ($R^{11}$ and $R^{12}$ each independently represent H or C1-6 alkyl)), haloalkylsulfanyl, haloalkylsulfinyl, haloalkylsulfonyl, C1-6 alkylthio, C1-6 alkylsulfinyl, C1-6 alkylsulfonyl, aminosulfonyl, sulfo, or sulfamoyl;
$R^5$ represents H, hydroxy, C1-6 alkyl, or halogen;
$R^6$ represents C1-6 alkyl;
$R^7$ represents boronic acid ($-B(OH)_2$), a boronic acid ester, or a boronic acid amide,
with the proviso that when $R^1$, $R^2$, $R^3$, and $R^4$ are all H, $R^5$ represents hydroxy or F, or when $R^1$, $R^2$, $R^3$, and $R^4$ are all H, $R^6$ represents C2-6 alkyl.

[2] The compound or a pharmaceutically acceptable salt thereof according to [1], wherein $R^6$ represents methyl or ethyl.
[3] The compound or a pharmaceutically acceptable salt thereof according to [1] or [2], wherein $R^7$ represents boronic acid ($B(OH)_2$) or a pinacol ester of boronic acid.
[4] The compound or a pharmaceutically acceptable salt thereof according to any one of [1] to [3], wherein $R^5$ represents H.
[5] The compound or a pharmaceutically acceptable salt thereof according to any one of [1] to [4], wherein any one or two or more of $R^1$, $R^2$, $R^3$, and $R^4$ are each independently Cl, F, C1-4 alkyl, C1-4 alkoxy, C1-4 alkylthio, $CH_2X$, $CHX_2$, or $CX_3$ (X represents F) .
[6] An L-type amino acid transporter 1 (LAT1) -selective agent including the compound or a pharmaceutically acceptable salt thereof according to any one of [1] to [5].
[7] An agent for BNCT, including the compound or a pharmaceutically acceptable salt thereof according to any one of [1] to [5].
[8] A diagnostic agent containing a radioisotope, the diagnostic agent including the compound or a pharmaceutically acceptable salt thereof according to any one of [1] to [5].
[9] A compound represented by the following formula (III) or a pharmaceutically acceptable salt thereof:

[Formula 2]

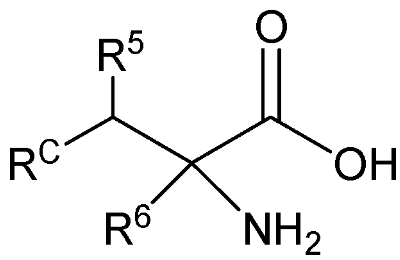

(III)

in the formula (III),

$R^c$ represents a heterocyclic ring having one group substituted with boronic acid ($-B(OH)_2$), a boronic acid ester, or a boronic acid amide and optionally substituted at 1 to 3 positions with halogen, hydroxy, cyano, C1-6 alkyl, C1-6 alkoxy, benzyloxy, C1-6 alkoxy C1-6 alkyl, nitro, C1-6 haloalkyl, aminocarbonyl, C1-C6 alkylaminocarbonyl ($CONR^8R^9$ ($R^8$ and $R^9$ each independently represent H or C1-6 alkyl)), C1-C6 alkoxycarbonyl, C1-C6 alkylcarbonyl, $COOR^{10}$ ($R^{10}$ represents H or C1-6 alkyl, amino, alkylamino ($NR^{11}R^{12}$ ($R^{11}$ and $R^{12}$ each independently represent H or C1-6 alkyl)), haloalkylsulfanyl, haloalkylsulfinyl, haloalkylsulfonyl, C1-6 alkylthio, C1-6 alkylsulfinyl, C1-6 alkylsulfonyl, aminosulfonyl, sulfo, or sulfamoyl;
the heterocyclic ring is one selected from the group consisting of pyridine, pyrimidine, thiophene, triazine, pyrrole, pyrazine, oxazole, isoxazole, oxadiazole, thiadiazole, isothiazole, and thiazole,
$R^5$ represents H, hydroxy, C1-6 alkyl, or halogen; and
$R^6$ represents C1-6 alkyl.

[10] A compound represented by the following formula (IV) or a pharmaceutically acceptable salt thereof:

[Formula 3]

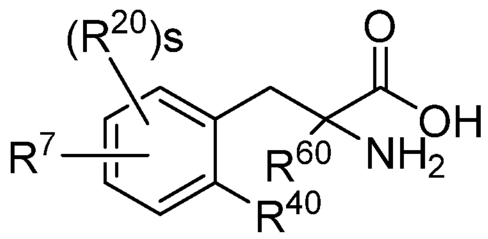

(IV)

in the formula (IV),

$R^{20}$ independently at each occurrence represents H, halogen, hydroxy, cyano, C1-6 alkyl, C1-6 alkoxy, benzyloxy, C1-6 alkoxy C1-6 alkyl, nitro, C1-6 haloalkyl, aminocarbonyl, C1-C6 alkylaminocarbonyl ($CONR^8R^9$ ($R^8$ and $R^9$ independently represent H or C1-6 alkyl)), C1-C6 alkoxycarbonyl, C1-C6 alkylcarbonyl, $COOR^{10}$ ($R^{10}$ represents H or C1-6 alkyl, amino, alkylamino ($NR^{11}R^{12}$ ($R^{11}$ and $R^{12}$ each independently represent H or C1-6 alkyl)), haloalkylsulfanyl, haloalkylsulfinyl, haloalkylsulfonyl, C1-6 alkylthio, C1-6 alkylsulfinyl, C1-6 alkylsulfonyl, aminosulfonyl, sulfo, or sulfamoyl;
$R^7$ represents boronic acid ($-B(OH)_2$), a boronic acid ester, or a boronic acid amide;
$R^{40}$ and $R^{60}$ together form a ring structure fused to a benzene ring, wherein the ring structure represents unsubstituted C5-7 cycloalkyl or unsubstituted C6-7 cycloalkenyl;
s represents any integer of 1 to 3,
with the proviso that

[Formula 4]

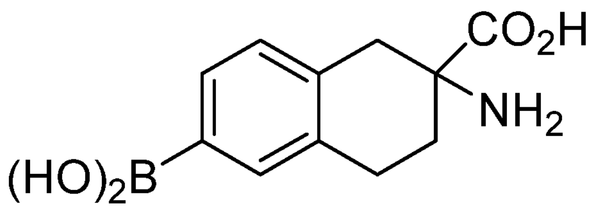

is excluded.

The novel compounds of the present invention are superior in uptake into LAT1 expressing cells and can be conveniently used for boron neutron capture therapy.

DETAILED DESCRIPTION OF THE PREFERRED EMBODIMENTS

In the present specification, when a compound having an asymmetric carbon is represented, the compound may be any of a racemate, an R-isomer, and an S-isomer unless otherwise indicated.

[4-Borono-phenylalanine derivatives]

In one aspect of the present invention, the borono-phenylalanine derivative is a 4-borono-phenylalanine derivative, and is a compound represented by the following formula (I) or a pharmaceutically acceptable salt thereof.

[Formula 5]

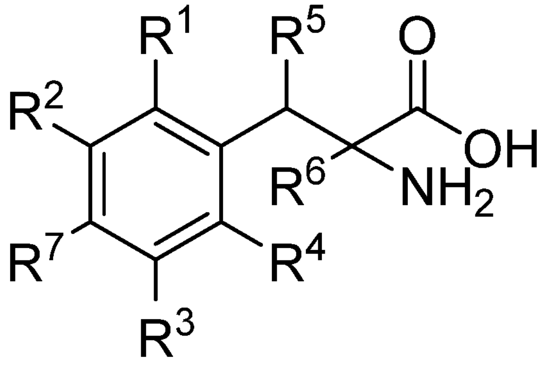

(I)

in the formula (I),

$R^1$, $R^2$, $R^3$, and $R^4$ each independently represent H, halogen, hydroxy, cyano, C1-6 alkyl, C1-6 alkoxy, benzyloxy, C1-6 alkoxy C1-6 alkyl, nitro, C1-6 haloalkyl, aminocarbonyl, C1-C6 alkylaminocarbonyl ($CONR^8R^9$ ($R^8$ and $R^9$ each independently represent H or C1-6 alkyl)), C1-C6 alkoxycarbonyl, C1-C6 alkylcarbonyl, $COOR^{10}$ ($R^{10}$ represents H or C1-6 alkyl, amino, alkylamino ($NR^{11}R^{12}$ ($R^{11}$ and $R^{12}$ each independently represent H or C1-6 alkyl)), haloalkylsulfanyl, haloalkylsulfinyl, haloalkylsulfonyl, C1-6 alkylthio, C1-6 alkylsulfinyl, C1-6 alkylsulfonyl, aminosulfonyl, sulfo, or sulfamoyl;
$R^5$ represents H, hydroxy, C1-6 alkyl, or halogen;
$R^6$ represents C1-6 alkyl;
$R^7$ represents boronic acid ($-B(OH)_2$), a boronic acid ester, or a boronic acid amide,
with the proviso that when $R^1$, $R^2$, $R^3$, and $R^4$ are all H, $R^5$ represents hydroxy or F, or when $R^1$, $R^2$, $R^3$, and $R^4$ are all H, $R^6$ represents C2-6 alkyl.

In the present specification, the halogen may be any of F, Cl, Br, and I, and is particularly preferably F, Cl, or Br.

In the present specification, the C1-C6 alkyl refers to a linear or branched C1-C6 saturated hydrocarbon group. This definition also includes those used in alkoxy, alkoxyalkyl, and haloalkyl. Examples of the C1-6 alkyl group include methyl, ethyl, propyl (e.g., n-propyl and isopropyl), butyl (e.g., n-butyl, isobutyl, and t-butyl), and pentyl (e.g., n-pentyl, isopentyl, and neopentyl) . The C1-6 alkyl group is preferably a linear or branched C1-C4 alkyl group, and examples thereof include, but are not limited to, a methyl group, an ethyl group, an isopropyl group, and a butyl group.

In the present specification, C1-C6 haloalkyl refers to a C1-C6 alkyl group having one or more halogen substituents. The haloalkyl group is preferably represented by $C_2X_5$, $CH_2X$, $CHX_2$, or $CX_3$ (X represents Cl, F, Br, or I), and examples thereof include, but are not limited to, $CF_3$, $C_2F_5$, $CHF_2$, $CCl_3$, $CHCl_2$, and $C_2Cl_5$.

In the present specification, C1-C6 alkoxy represents a group having a linear or branched C1-C6 alkyl group and an oxygen atom. C1-C6 alkoxy preferably has a linear or branched C1-C4 alkyl, and examples thereof include, but are not limited to, methoxy, ethoxy, isopropoxy, and butoxy.

In the present specification, examples of C1-6 alkoxy C1-6 alkyl include, but are not limited to, methoxyethyl and ethoxyethyl.

In the present specification, examples of C1-C6 alkylaminocarbonyl ($CONR^8R^9$ ($R^8$ and $R^9$ each independently represent H or C1-6 alkyl)) include, but are not limited to, methylaminocarbonyl, dimethylaminocarbonyl, ethylaminocarbonyl, and i-propylaminocarbonyl.

In the present specification, examples of C1-C6 alkylcarbonyl include, but are not limited to, methylcarbonyl and ethylcarbonyl.

In the present specification, examples of C1-C6 alkoxycarbonyl include, but are not limited to, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, and hexyloxycarbonyl.

In the present specification, examples of $COOR^{10}$ ($R^{10}$ is H or C1-6 alkyl, amino, alkylamino ($NR^{11}$ $R^{12}$ ($R^{11}$ and $R^{12}$ each independently represent H or C1-6 alkyl)) include, but are not limited to, methyloxycarbonyl and aminooxycarbonyl.

In the present specification, examples of haloalkylsulfanyl, haloalkylsulfinyl, and haloalkylsulfonyl include, but are not limited to, trifluoromethylsulfanyl, a trifluoromethylsulfinyl group, and trifluoromethylsulfonyl.

In the present specification, examples of C1-C6 alkylthio include, but are not limited to, methylthio, ethylthio, propylthio, isopropylthio, butylthio, sec-butylthio, tert-butylthio, pentylthio, and hexylthio.

In the present specification, examples of C1-C6 alkylsulfinyl include, but are not limited to, methylsulfinyl and ethylsulfinyl.

In the present specification, examples of C1-C6 alkylsulfonyl include, but are not limited to, methylsulfonyl and ethylsulfonyl.

In the derivatives of the present invention, $R^7$ represents any of a boronic acid group ($-B(OH)_2$), a boronic acid ester group, or a boronic acid amide group: for example, the boronic acid ester group and the boronic acid amide group in this definition represent a group having a chain structure such as $-B(NR^{41})_2$ or $-B(OR^{41})_2$ at the position of $R^7$ or a group having a cyclic structure together with an atom B at the position of $R^7$. Here, $R^{41}$ represents a linear or branched C1-C10 alkyl group. Here, the "linear or branched C1-C10 alkyl group" may be any alkyl group having 1-10 carbon atoms. The alkyl group is preferably a linear or branched C1-C8 alkyl group, and more preferably a linear or branched C1-C6 alkyl group. Examples of these groups include, but are not limited to, a methyl group, an ethyl group, an isopropyl group, and a butyl group. Furthermore, in the cyclic structure referred to herein, the atom that is interposed is not necessarily only an O atom, and may be an N atom. Examples thereof include, but are not limited to, groups composed of an ester or ester analog composed of an atom B and any selected from the group consisting of pinacol, 2,2-dimethyl-1,3-propanediol, N-methyldiethanolamine, 1-6-diaminonaphthalene, N-methyliminodiacetic acid, 1,1,1-trishydroxymethylethane, and catechol. Examples thereof include, but are not limited to, a boronic acid pinacol ester, a boronic acid MIDA ester, a boronic acid 1, 3-propanediol ester, a boronic acid neopentyl glycol ester, a boronic acid catechol ester, a boronic acid pinanediol ester, a boronic acid biscyclohexyldiol ester, a boronic acid MPM ester, a trifluoroborate salt, a cyclic triol borate salt, and a cyclic compound formed of diaminonaphthalenamide and boron.

Among them, $R^7$ is particularly preferably boronic acid or a boronic acid ester having a chain or cyclic structure, and most preferably boronic acid.

Here, as to boron atoms, the proportion of boron-10 is not limited, but may be preferably 75% by mass or more, more preferably 80% by mass or more, even more preferably 90% by mass or more, and particularly preferably 95% by mass or more.

Natural boron (boron) includes boron-10 and boron-11 as isotopes in the proportions of 20% boron-10 to 80% boron-11. Therefore, prior to the production of the 4-borono-phenylalanine derivative of the present invention, it is also preferable to concentrate boron having a mass number of 10 (boron-10). In the present invention, for example, a commercially available product may be used as the boron atom source. As such a commercially available product, for example, 10B concentrated boric acid (manufactured by Stella Chemifa Corporation) can be used.

Here, boron-10 can be measured by multi-type ICP-optical emission spectrometry (ICP-OES) using Agilent 710 (manufactured by Agilent Technologies Inc.) . The ICP-OES used for the measurement is adjusted in accordance with JIS K 0116.

In the compound, it is particularly preferable that any one or two or more of $R^1$, $R^2$, $R^3$, and $R^4$ are each independently, but are not limited to, Cl, F, C1-3 alkyl, C1-3 alkoxy, $CH_2F$, $CHF_2$, $CF_3$, or C1-3 alkylthio.

In the compound, $R^5$ particularly preferably represents, but is not limited to, H. At this time, however, it is impermissible that all of $R^1$, $R^2$, $R^3$, and $R^4$ are H.

In the compound, $R^6$ particularly preferably represents, but is not limited to, methyl or ethyl.

In the compound, $R^7$ is particularly preferably, but is not limited to, boronic acid ($B(OH)_2$) or a pinacol ester of boronic acid.

In one aspect of the present invention, it is preferable that in the compound, any one or two or more of $R^1$, $R^2$, $R^3$, and $R^4$ are each independently Cl or F, $R^5$ represents H, $R^6$ represents methyl, and $R^7$ represents boronic acid ($B(OH)_2$).

In another aspect of the present invention, it is preferable that in the compound, any one or two or more of $R^1$, $R^2$, $R^3$, and $R^4$ are each independently $CH_2F$, $CHF_2$, or $CF_3$, $R^5$ represents H, $R^6$ represents methyl, and $R^7$ represents boronic acid ($B(OH)_2$).

In still another aspect of the present invention, it is preferable that in the compound, any one or two or more of $R^1$, $R^2$, $R^3$, and $R^4$ are each independently methyl or ethyl, $R^5$ represents H, $R^6$ represents methyl, and $R^7$ represents boronic acid ($B(OH)_2$).

In still another aspect of the present invention, it is preferable that in the compound, any one or two or more of $R^1$, $R^2$, $R^3$, and $R^4$ are each independently methoxy or ethoxy, $R^5$ represents H, $R^6$ represents methyl, and $R^7$ represents boronic acid ($B(OH)_2$).

In still another aspect of the present invention, it is preferable that in the compound, any one or two or more of $R^1$, $R^2$, $R^3$, and $R^4$ are each independently methylthio or ethylthio, $R^5$ represents H, $R^6$ represents methyl, and $R^7$ represents boronic acid ($B(OH)_2$).

In still another aspect of the present invention, it is preferable that in the compound, all of $R^1$, $R^2$, $R^3$, and $R^4$ are H, $R^5$ represents H, $R^6$ represents ethyl, and $R^7$ represents boronic acid ($B(OH)_2$).

Examples of the "pharmaceutically acceptable salt" as used in the present invention includes a salt with an inorganic base, a salt with an organic base, a salt with an inorganic acid, a salt with an organic acid, and a salt with a

basic or acidic amino acid. Suitable examples of the salt with an inorganic base include alkali metal salts such as sodium salts and potassium salts; alkaline earth metal salts such as calcium salts and magnesium salts; aluminum salts and ammonium salts. Suitable examples of the salt with an organic base include salts with trimethylamine, triethylamine, pyridine, picoline, ethanolamine, diethanolamine, triethanolamine, dicyclohexylamine, and N,N'-dibenzylethylenediamine. Suitable examples of the salt with an inorganic acid include salts with hydrochloric acid, hydrobromic acid, nitric acid, sulfuric acid, and phosphoric acid. Suitable examples of the salt with an organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid. Suitable examples of the salt with a basic amino acid include salts with arginine, lysine, and ornithine, and suitable examples of the salt with an acidic amino acid include salts with aspartic acid and glutamic acid.

[Borono-phenylalanine derivative having a heterocyclic ring]

Another aspect of the present invention relates to a compound represented by the following formula (III) or a pharmaceutically acceptable salt thereof.

[Formula 6]

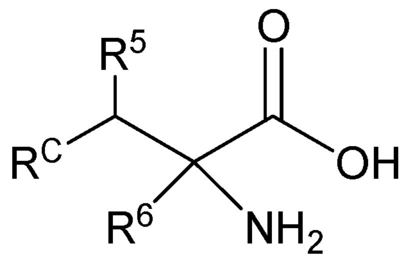

(III)

in the formula (III),

$R^c$ represents a heterocyclic ring having one group substituted with boronic acid ($-B(OH)_2$), a boronic acid ester, or a boronic acid amide and optionally substituted at 1 to 3 positions with halogen, hydroxy, cyano, C1-6 alkyl, C1-6 alkoxy, benzyloxy, C1-6 alkoxy C1-6 alkyl, nitro, C1-6 haloalkyl, aminocarbonyl, C1-C6 alkylaminocarbonyl ($CONR^8R^9$ ($R^8$ and $R^9$ each independently represent H or C1-6 alkyl)), C1-C6 alkoxycarbonyl, C1-C6 alkylcarbonyl, $COOR^{10}$ ($R^{10}$ represents H or C1-6 alkyl, amino, alkylamino ($NR^{11}R^{12}$ ($R^{11}$ and $R^{12}$ each independently represent H or C1-6 alkyl)), haloalkylsulfanyl, haloalkylsulfinyl, haloalkylsulfonyl, C1-6 alkylthio, C1-6 alkylsulfinyl, C1-6 alkylsulfonyl, aminosulfonyl, sulfo, or sulfamoyl,
the heterocyclic ring is one selected from the group consisting of pyridine, pyrimidine, thiophene, triazine, pyrrole, pyrazine, oxazole, isoxazole, oxadiazole, thiadiazole, isothiazole, and thiazole,
$R^5$ represents H, hydroxy, C1-6 alkyl, or halogen; and
$R^6$ represents C1-6 alkyl.

Here, the conditions including the type of substituent, suitable examples, the type of salt, and the like are as described in the section of [4-Borono-phenylalanine derivatives].

[Borono-phenylalanine derivatives having fused ring]

Yet another aspect of the present invention relates to a compound represented by the following formula (IV) or a pharmaceutically acceptable salt thereof.

[Formula 7]

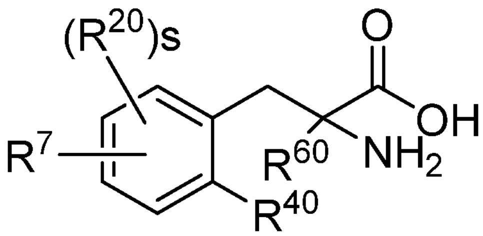

(IV)

in the formula (IV),

$R^{20}$ independently at each occurrence represents H, halogen, hydroxy, cyano, C1-6 alkyl, C1-6 alkoxy, benzyloxy, C1-6 alkoxy C1-6 alkyl, nitro, C1-6 haloalkyl, aminocarbonyl, C1-C6 alkylaminocarbonyl ($CONR^8R^9$ ($R^8$ and $R^9$ independently represent H or C1-6 alkyl)), C1-C6 alkoxycarbonyl, C1-C6 alkylcarbonyl, $COOR^{10}$ ($R^{10}$ represents H or C1-6 alkyl, amino, alkylamino ($NR^{11}R^{12}$ ($R^{11}$ and $R^{12}$ each independently represent H or C1-6 alkyl)), haloalkylsulfanyl, haloalkylsulfinyl, haloalkylsulfonyl, C1-6 alkylthio, C1-6 alkylsulfinyl, C1-6 alkylsulfonyl, aminosulfonyl, sulfo, or sulfamoyl;
$R^7$ represents boronic acid ($-B(OH)_2$), a boronic acid ester, or a boronic acid amide;
$R^{40}$ and $R^{60}$ together form a ring structure fused to a benzene ring, wherein the ring structure represents unsubstituted C5-7 cycloalkyl or unsubstituted C6-7 cycloalkenyl;
s represents any integer of 1 to 3,
with the proviso that

[Formula 8]

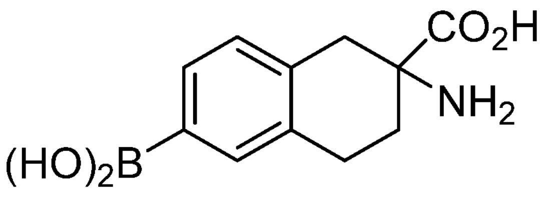

is excluded.

Here, among such compounds, a compound represented by the following chemical formula may be particularly preferable.

[Formula 9]

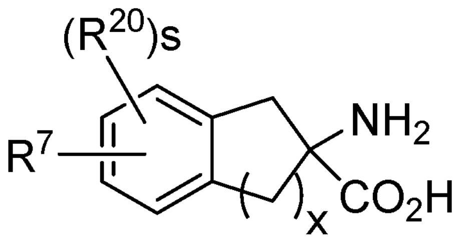

(IV)

in the formula (IV),

$R^{20}$ independently at each occurrence represents H, halogen, hydroxy, cyano, C1-6 alkyl, C1-6 alkoxy, benzyloxy, C1-6 alkoxy C1-6 alkyl, nitro, C1-6 haloalkyl, aminocarbonyl, C1-C6 alkylaminocarbonyl ($CONR^8R^9$ ($R^8$ and $R^9$ independently represent H or C1-6 alkyl)), C1-C6 alkoxycarbonyl, C1-C6 alkylcarbonyl, $COOR^{10}$ ($R^{10}$ represents H or C1-6 alkyl, amino, alkylamino ($NR^{11}R^{12}$ ($R^{11}$ and $R^{12}$ each independently represent H or C1-6 alkyl)), haloalkylsulfanyl, haloalkylsulfinyl, haloalkylsulfonyl, C1-6 alkylthio, C1-6 alkylsulfinyl, C1-6 alkylsulfonyl, aminosulfonyl, sulfo, or sulfamoyl;

$R^7$ represents boronic acid ($-B(OH)_2$), a boronic acid ester, or a boronic acid amide;

s represents any integer of 1 to 3; and

x represents any integer of 1 to 3,

with the proviso that

[Formula 10]

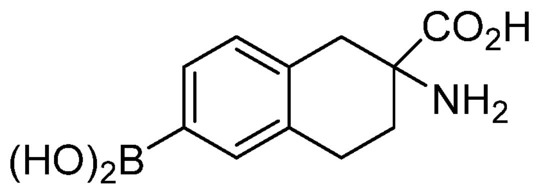

is excluded.

In the present specification, the halogen may be any of F, Cl, Br, and I, and is particularly preferably F, Cl, or Br.

In the present specification, the C1-C6 alkyl refers to a linear or branched C1-C6 saturated hydrocarbon group. This definition also includes those used in alkoxy, alkoxyalkyl, and haloalkyl. Examples of the C1-6 alkyl group include methyl, ethyl, propyl (e.g., n-propyl and isopropyl), butyl (e.g., n-butyl, isobutyl, and t-butyl), and pentyl (e.g., n-pentyl, isopentyl, and neopentyl) . The C1-6 alkyl group is preferably a linear or branched C1-C4 alkyl group, and examples thereof include, but are not limited to, a methyl group, an ethyl group, an isopropyl group, and a butyl group.

In the present specification, C1-C6 haloalkyl refers to a C1-C6 alkyl group having one or more halogen substituents. The haloalkyl group is preferably represented by $C_2X_5$, $CH_2X$, $CHX_2$, or $CX_3$ (X represents Cl, F, Br, or I), and examples thereof include, but are not limited to, $CF_3$, $C_2F_5$, $CHF_2$, $CCl_3$, $CHCl_2$, and $C_2Cl_5$.

In the present specification, C1-C6 alkoxy represents a group having a linear or branched C1-C6 alkyl group and an oxygen atom. C1-C6 alkoxy preferably has a linear or branched C1-C4 alkyl, and examples thereof include, but are not limited to, methoxy, ethoxy, isopropoxy, and butoxy.

In the present specification, examples of C1-6 alkoxy C1-6 alkyl include, but are not limited to, methoxyethyl and ethoxyethyl.

In the present specification, examples of C1-C6 alkylaminocarbonyl ($CONR^8R^9$ ($R^8$ and $R^9$ each independently represent H or C1-6 alkyl)) include, but are not limited to, methylaminocarbonyl, dimethylaminocarbonyl, ethylaminocarbonyl, and i-propylaminocarbonyl.

In the present specification, examples of C1-C6 alkylcarbonyl include, but are not limited to, methylcarbonyl and ethylcarbonyl.

In the present specification, examples of C1-C6 alkoxycarbonyl include, but are not limited to, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, and hexyloxycarbonyl.

In the present specification, examples of $COOR^{10}$ ($R^{10}$ is H or C1-6 alkyl, amino, alkylamino ($NR^{11}$ $R^{12}$ ($R^{11}$ and $R^{12}$ each independently represent H or C1-6 alkyl)) include, but are not limited to, methyloxycarbonyl and aminooxycarbonyl.

In the present specification, examples of haloalkylsulfanyl, haloalkylsulfinyl, and haloalkylsulfonyl include, but are not limited to, trifluoromethylsulfanyl, a trifluoromethylsulfinyl group, and trifluoromethylsulfonyl, respectively.

In the present specification, examples of C1-C6 alkylthio include, but are not limited to, methylthio, ethylthio, propylthio, isopropylthio, butylthio, sec-butylthio, tert-butylthio, pentylthio, and hexylthio.

In the present specification, examples of C1-C6 alkylsulfinyl include, but are not limited to, methylsulfinyl and ethylsulfinyl.

In the present specification, examples of C1-C6 alkylsulfonyl include, but are not limited to, methylsulfonyl and ethylsulfonyl.

In the present specification, examples of the unsubstituted C5-7 cycloalkyl include cyclopentyl, cyclohexyl, and cycloheptyl.

In the present specification, examples of the unsubstituted C6-7 cycloalkenyl include cyclohexenyl and cycloheptenyl.

In the derivatives of the present invention, $R^7$ represents any of a boronic acid group ($-B(OH)_2$), a boronic acid ester group, or a boronic acid amide group: for example, the boronic acid ester group and the boronic acid amide group in this definition represent a group having a chain structure such as $-B(NR^{41})_2$ or $-B(OR^{41})_2$ at the position of $R^7$ or a group having a cyclic structure together with an atom B at the position of $R^7$. Here, $R^{41}$ represents a linear or branched C1-C10 alkyl group. Here, the "linear or branched C1-C10 alkyl group" may be any alkyl group having 1-10 carbon atoms. The alkyl group is preferably a linear or branched C1-C8 alkyl group, and more preferably a linear or branched C1-C6 alkyl group. Examples of these groups include, but are not limited to, a methyl group, an ethyl group, an isopropyl group, and a butyl group. Furthermore, in the cyclic structure referred to herein, the atom that is interposed is not necessarily only an O atom, and may be an N atom. Examples thereof include, but are not limited to, groups composed of an ester or ester analog composed of an atom B and any selected from the group consisting of pinacol, 2,2-dimethyl-1,3-propanediol, N-methyldiethanolamine, 1-6-diaminonaphthalene, N-methyliminodiacetic acid, 1,1,1-trishydroxymethylethane, and catechol. Examples thereof include, but are not limited to, a boronic acid pinacol ester, a boronic acid MIDA ester, a boronic acid 1, 3-propanediol ester, a boronic acid neopentyl glycol ester, a boronic acid catechol ester, a boronic acid pinanediol ester, a boronic acid biscyclohexyldiol ester, a boronic acid MPM ester, a trifluoroborate salt, a cyclic triol borate salt, and a cyclic compound formed of diaminonaphthalenamide and boron.

Among them, $R^7$ is particularly preferably boronic acid or a boronic acid ester having a chain or cyclic structure, and most preferably boronic acid.

Here, as to boron atoms, the proportion of boron-10 is not limited, but may be preferably 75% by mass or more, more preferably 80% by mass or more, even more preferably 90% by mass or more, and particularly preferably 95% by mass or more.

Natural boron (boron) includes boron-10 and boron-11 as isotopes in the proportions of 20% boron-10 to 80% boron-11. Therefore, prior to the production of the 4-borono-phenylalanine derivative of the present invention, it is also preferable to concentrate boron having a mass number of 10 (boron-10). In the present invention, for example, a commercially available product may be used as the boron atom source. As such a commercially available product, for example, 10B concentrated boric acid (manufactured by Stella Chemifa Corporation) can be used.

Here, boron-10 can be measured by multi-type ICP-optical emission spectrometry (ICP-OES) using Agilent 710 (manufactured by Agilent Technologies Inc.) . The ICP-OES used for the measurement is adjusted in accordance with JIS K 0116.

It is particularly preferable that in the compound, any one to three of $R^{20}$ are each independently, but are not limited to, H, Cl, F, C1-3 alkyl, C1-3 alkoxy, $CH_2F$, $CHF_2$, $CF_3$, or C1-3 alkylthio.

In the compound, $R^7$ is particularly preferably, but is not limited to, boronic acid ($B(OH)_2$) or a pinacol ester of boronic acid.

In one aspect of the present invention, it is preferable that in the compound, any one to three of $R^{20}$ are each independently Cl or F, x is 1 to 3, and $R^7$ represents boronic acid ($B(OH)_2$).

In another aspect of the present invention, it is preferable that in the compound, any one to three of $R^{20}$ are each independently $CH_2F$, $CHF_2$, or $CF_3$, x is 1 to 2, and $R^7$ represents boronic acid ($B(OH)_2$).

In still another aspect of the present invention, it is preferable that in the compound, any one to three of $R^{20}$ are each independently methyl or ethyl, x is 1 to 2, and $R^7$ represents boronic acid ($B(OH)_2$).

In still another aspect of the present invention, it is preferable that in the compound, any one or two or more of $R^1$, $R^2$, $R^3$, and $R^4$ are each independently methoxy or ethoxy, $R^5$ represents H, $R^6$ represents methyl, and $R^7$ represents boronic acid ($B(OH)_2$).

In still another aspect of the present invention, it is preferable that in the compound, any one to three of $R^{20}$ are each independently methylthio or ethylthio, x is 1 to 3, and $R^7$ represents boronic acid ($B(OH)_2$).

Examples of the "pharmaceutically acceptable salt" as used in the present invention includes a salt with an inorganic base, a salt with an organic base, a salt with an inorganic acid, a salt with an organic acid, and a salt with a basic or acidic amino acid. Suitable examples of the salt with an inorganic base include alkali metal salts such as sodium salts and potassium salts; alkaline earth metal salts such as calcium salts and magnesium salts; aluminum salts and ammonium salts. Suitable examples of the salt with an organic base include salts with trimethylamine, triethylamine, pyridine, picoline, ethanolamine, diethanolamine, triethanolamine, dicyclohexylamine, and N,N'-dibenzylethylenediamine. Suitable examples of the salt with an inorganic acid include salts with hydrochloric acid, hydrobromic acid, nitric acid, sulfuric acid, and phosphoric acid. Suitable examples of the salt with an organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid. Suitable examples of the salt with a basic amino acid include salts with arginine, lysine, and ornithine, and suitable examples of the salt with an acidic amino acid include salts with aspartic acid and glutamic acid.

[Particularly preferable compounds of the present invention]

Among the borono-phenylalanine derivatives of the present invention, one selected from the group consisting of the following compounds or a salt thereof is particularly preferable.

(S)-2-amino-3-(4-borono-2-fluorophenyl)-2-methylpropa noic acid;
(R)-2-amino-3-(4-borono-2-fluorophenyl)-2-methylpropa noic acid;
(S)-2-amino-3-(4-borono-2-methylphenyl)-2-methylpropa noic acid;
(R)-2-amino-3-(4-borono-2-methylphenyl)-2-methylpropa noic acid;
(S)-2-amino-3-(4-borono-2-(trifluoromethyl)phenyl)-2-methylpropanoic acid;
(R)-2-amino-3-(4-borono-2-(trifluoromethyl)phenyl)-2-methylpropanoic acid;
(S)-2-amino-3-(4-borono-2-methoxyphenyl)-2-methylprop anoic acid;
(R)-2-amino-3-(4-borono-2-methoxyphenyl)-2-methylprop anoic acid;
(S)-2-amino-3-(4-borono-2-chlorophenyl)-2-methylpropa noic acid;
(R)-2-amino-3-(4-borono-2-chlorophenyl)-2-methylpropa noic acid;
(S)-2-amino-3-(4-borono-2,6-difluorophenyl)-2-methylp ropanoic acid;
(R)-2-amino-3-(4-borono-2,6-difluorophenyl)-2-methylp ropanoic acid;
(S)-2-amino-3-(4-borono-5-chloro-2-fluorophenyl)-2-me thylpropanoic acid;
(R)-2-amino-3-(4-borono-5-chloro-2-fluorophenyl)-2-me thylpropanoic acid;
(S)-2-amino-3-(4-borono-2,3-difluorophenyl)-2-methylp ropanoic acid;
(R)-2-amino-3-(4-borono-2,3-difluorophenyl)-2-methylp ropanoic acid;
(S)-2-amino-3-(4-borono-3-fluorophenyl)-2-methylpropa noic acid;
(R)-2-amino-3-(4-borono-3-fluorophenyl)-2-methylpropa noic acid;
(S)-2-amino-3-(4-borono-3-methylphenyl)-2-methylpropa noic acid;
(R)-2-amino-3-(4-borono-3-methylphenyl)-2-methylpropa noic acid;
(S)-2-amino-2-(4-boronobenzyl)butanoic acid;
(R)-2-amino-2-(4-boronobenzyl)butanoic acid;
(S)-2-amino-3-(4-borono-2-cyanophenyl)-2-methylpropan oic acid;
(R)-2-amino-3-(4-borono-2-chloro-5-fluorophenyl)-2-me thylpropanoic acid;
(S)-2-amino-3-(4-borono-2-chloro-5-fluorophenyl)-2-me thylpropanoic acid;
(S)-2-amino-3-(4-borono-2-nitrophenyl)-2-methylpropan oic acid;
(S)-2-amino-3-(4-borono-3-nitrophenyl)-2-methylpropan oic acid;
(S)-2-amino-3-(3-amino-4-boronophenyl)-2-methylpropan oic acid;
(S)-2-amino-3-(2-amino-4-boronophenyl)-2-methylpropan oic acid;
(S)-2-amino-3-(4-borono-2-(methylthio)phenyl)-2-methy lpropanoic acid;
(S)-2-amino-3-(4-borono-2-(difluoromethyl)phenyl)-2-m ethylpropanoic acid;
(R)-2-amino-3-(4-borono-2-(difluoromethyl)phenyl)-2-m ethylpropanoic acid;
(S)-2-amino-3-(4-borono-3-chlorophenyl)-2-methylpropa noic acid;
(S)-2-amino-3-(5-boronopyridin-2-yl)propanoic acid;
(S)-2-amino-3-(5-borono-3-fluoropyridin-2-yl)propanoi c acid;
(S)-2-amino-3-(5-borono-3-methylpyridin-2-yl)propanoi c acid;
(S)-2-amino-3-(5-borono-3-chloropyridin-2-yl)propanoi c acid; or
(S)-2-amino-3-(5-borono-4-methylpyridin-2-yl)propanoi c acid.

[Method for producing 4-borono-phenylalanine derivative]

In the present invention, the method for producing a novel 4-borono-phenylalanine derivative is not limited, and an ordinary amino acid synthesis method is used. A particularly preferable method may be, but is not limited to, the following method, for example.

First, a protected amino acid is reacted with an organic halide represented by the following general formula (II) in the presence of an organic solvent, a basic aqueous solution, and a phase transfer catalyst.

[Formula 11]

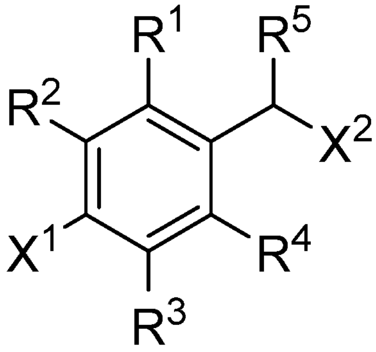

(II)

In the formula (II),

$R^1$, $R^2$, $R^3$, and $R^4$ each independently represent H, halogen, hydroxy, cyano, C1-6 alkyl, C1-6 alkoxy, benzyloxy, C1-6 alkoxy C1-6 alkyl, nitro, C1-6 haloalkyl, aminocarbonyl, C1-C6 alkylaminocarbonyl, C1-C6 alkoxycarbonyl, C1-C6 alkylcarbonyl, $COOR^{10}$ ($R^{10}$ represents H or C1-6 alkyl, amino, alkylamino), haloalkylsulfanyl, haloalkylsulfinyl, a haloalkylsulfonyl group, C1-6 alkylthio, C1-6 alkylsulfinyl, C1-6 alkylsulfonyl, aminosulfonyl, sulfo, or sulfamoyl;
$R^5$ represents H, hydroxy, C1-6 alkyl, or halogen; and
$X^1$ and $X^2$ each independently represent halogen (F, Cl, Br, I).

Here, as the organic halide represented by the formula (II), a compound prepared by a known method can be used as it is, or a commercially available product can be used.

Among them, for example, 4-iodobenzyl bromide and 4-bromo-2-fluorobenzyl bromide are available from Tokyo Chemical Industry Co., Ltd. or Combi-Blocks Inc. 4-Bromo-3-fluorobenzyl bromide, 1-bromo-4-(bromomethyl)-2,3-difluorobenzene, 4-bromo-1-(bromomethyl)-2-methoxybenzene, 4-bromo-1-(bromomethyl)-2-nitrobenzene, 4-bromo-1-(bromomethyl)-2-chlorobenzene, 4-bromo-1-(bromomethyl)-2-(trifluoromethyl)benzene, 4-bromo-1-(bromomethyl)-2-(trifluoromethyl)benzene, 1-bromo-4-(bromomethyl)-2-methylbenzene are available from Combi-Blocks Inc. 4-Bromo-1-(bromomethyl)-2-methylbenzene and 4-bromo-2,6-difluorobenzyl bromide are available from Fluorochem Ltd.

5-Bromo-2-(bromomethyl)benzonitrile can be prepared, for example, from 5-bromo-2-methylbenzonitrile available from Tokyo Chemical Industry Co., Ltd., 1-bromo-4-(bromomethyl)-2-chloro-5-fluorobenzene can be prepared from 4-bromo-5-chloro-2-fluorotoluene available from Combi-Blocks Inc., and 4-bromo-1-(bromomethyl)-2-(methylthio)benzene can be prepared from 5-bromo-2-methylthioanisole available from Combi-Blocks Inc. These compounds can be prepared, for example, by allowing N-bromosuccinimide to act on commercially available compounds in the presence of 2,2'-azobis(isobutyronitrile).

4-(Bromomethyl)-1-iodo-2-nitrobenzene can be prepared, for example, by brominating (4-iodo-3-nitro-phenyl)-methanol available from Combi-Blocks Inc. with hydrobromic acid.

Other benzyl bromides are commercially available, and those unavailable can be prepared, for example, by allowing N-bromosuccinimide to act on toluene having a corresponding substituent in the presence of 2,2'-azobis(isobutyronitrile), or by reducing benzaldehyde or methyl benzoate having a corresponding substituent with sodium borohydride or lithium aluminum hydride to obtain benzyl alcohol, and then brominating the benzyl alcohol with hydrobromic acid or phosphorus tribromide.

The reaction between the organic halide represented by the formula (II) and the protected amino acid can proceed in the presence of an organic solvent, a basic aqueous solution, and a phase transfer catalyst. Examples of the protected amino acid include p-chlorobenzaldehyde imine and benzophenone imine. Preferably, the protected amino acid may be p-chlorobenzaldehyde imine having the following structure.

[Formula 12]

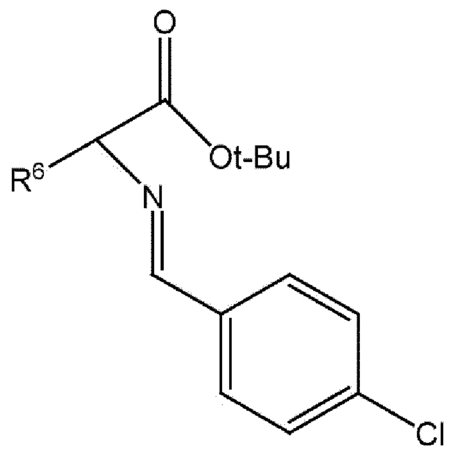

Here, suitable examples of the organic solvent to be used include, but are not limited to, toluene, benzene, xylene, mesitylene, ethyl ether, isopropyl ether, tetrahydrofuran, dioxane, ethyl acetate, isopropyl acetate, cyclopentyl methyl ether, and methyl t-butyl ether.

The basic aqueous solution is preferably an aqueous solution of calcium hydroxide, cesium hydroxide, potassium hydroxide, or the like.

The phase transfer catalyst may be, for example, a Maruoka's reagent. The Maruoka's reagent is not limited, and (R)-4,4-dibutyl-2,6-bis(3,4,5-trifluorophenyl)-4,5-dihydro-3H-dinaphtho[2,1-c:1',2'-e]azepinium bromide, (R)-4,4-dibutyl-2,6-bis(3,4,5-trifluorophenyl)-4,5-dihydro-3H-dinaphtho[2,1-c:1',2'-e]azepinium bromide, (S)-4,4-dibutyl-2,6-bis(3,4,5-trifluorophenyl)-4,5-dihydro-3H-dinaphtho[2,1-c:1',2'-e]azepinium bromide, (S)-4,4-dibutyl-2,6-bis(3,4,5-trifluorophenyl)-4,5-dihydro-3H-dinaphtho[2,1-c:1',2'-e]azepinium bromide, or the like can be preferably used.

The reaction temperature for this reaction is preferably between -20°C and 10°C, and the reaction time may be about 1 hour to about 60 hours.

After completion of the reaction, the mixture may be extracted with an organic solvent such as toluene, and optionally subjected to washing, drying, and filtration steps.

Next, to the reaction product is added a solvent, and the reaction product is reacted with an acid. Here, it is preferable to use an ether-based solvent as the solvent. Here, examples of the ether-based solvent include, but are not limited to, diethyl ether, tetrahydrofuran (THF), 2-methyltetrahydrofuran, dioxane, cyclopentyl methyl ether, glyme, and diglyme. In the present invention, tetrahydrofuran is particularly preferably used.

Examples of the acid include organic acids such as citric acid, acetic acid, trichloroacetic acid, trifluoroacetic acid, trifluoromethanesulfonic acid, toluenesulfonic acid, and methanesulfonic acid, and inorganic acids such as hydrochloric acid, sulfuric acid, nitric acid, and phosphoric acid.

The reaction is carried out at a temperature within the range of from 0°C to 50°C.

The reaction time is about 1 to about 10 hours, more preferably 2 to 8 hours, and still more preferably 3 to 6 hours.

The amino group of the resulting compound is protected by a conventional method. The protecting group is not limited, but for example, a carbamate-based protecting group, an amide-based protecting group, or an alkyl protecting group is preferably used. Examples of the carbamate-based protecting group include tert-butoxycarbonyl group (Boc), benzyloxycarbonyl group (Cbz), 9-fluorenylmethyloxycarbonyl group (Fmoc), and 2,2,2-trichloroethoxycarbonyl group (Troc); examples of the amide-based protecting group include acetyl group and benzoyl group; and examples of the alkyl protecting group include benzyl group.

Next, the compound obtained is reacted with a boron compound in the presence of a palladium catalyst, an organophosphorus compound, and a base in a solvent.

Here, examples of the palladium catalyst include, but are not limited to, palladium (II) acetate, palladium (II) chloride, tris(dibenzylideneacetone)dipalladium(0), [1,1'-bis(diphenylphosphino)ferrocene]palladium(II) dichloride dichloromethane adduct, and tetrakis(triphenylphosphine)palladium (0).

Examples of the organophosphorus compound include, but are not limited to, triphenylphosphine, tricyclohexylphosphine, 1,1'-bis(diphenylphosphino)ferrocene, 2-dicyclohexylphosphino-2',6'-dimethoxybiphenyl, 2-dicyclohexylphosphino-2',4',6'-triisopropylbiphenyl, and 2-dicyclohexylphosphino-2'-(N,N-dimethylamino)biphenyl.

Examples of the base include, but are not limited to, potassium acetate, sodium acetate, sodium carbonate,

cesium carbonate, potassium carbonate, and sodium hydrogen carbonate.

Examples of the boron compound include a boric acid ester or a boric acid amide, and the boron compound preferably refers to a compound represented by $B(OR)_3$, $B(NR)_3$, $B(OR)_2(NR)$, $(RO)_2B-B(OR)_2$, or $B(OR)(NR)_2$ (R represents a linear or branched C1-C10 alkyl group, a phenyl group, or a benzyl group), or the like. Among these, a compound represented by $(RO)_2B-B(OR)_2$ is particularly preferably used. The "linear or branched C1-C10 alkyl group" as referred to herein may be any alkyl group having 1 to 10 carbon atoms, and is preferably a linear or branched C1-C8 alkyl group, and more preferably a linear or branched C1-C6 alkyl group. Examples of these groups include, but are not limited to, a methyl group, an ethyl group, an isopropyl group, and a butyl group. Examples of the boron compound typically include, but are not limited to, bis(pinacolato)diboron.

Examples of the solvent include, but are not limited to, ether-based solvents such as 1,4-dioxane, tetrahydrofuran, and 1,2-dimethoxyethane; hydrocarbon-based solvent such as toluene; and polar solvents such as N,N-dimethylformamide and dimethyl sulfoxide. Preferred solvents include dimethyl sulfoxide. The reaction temperature is, for example, 20°C to 160°C, and preferably 60°C to 120°C.

Next, the compound obtained is sequentially deprotected, and thus a desired compound can be produced. Deprotection is performed according to a conventional method, and can be performed by, for example, hydrolysis, catalytic hydrogenation, decarboxylation, or oxidation.

In each step of the production method, purification is performed according to a conventional method, and can be appropriately modified.

In particular, when the compound is a racemate, it can be used as it is, or for example, the optical purity of an R-isomer or an S-isomer may be increased in order to obtain a preferred compound for use for boron neutron capture therapy.

The optical resolution may be performed using an appropriate known method, and for example, in addition to a method in which the optical resolution (using α-chymotrypsin or the like) is performed through a hydrolysis step and an esterification step, a simplified method including a simplified step in which an acylase is used through a hydrolysis step may be adopted.

[Method for producing borono-phenylalanine derivative having fused ring]

In the present invention, the method for producing a novel borono-phenylalanine derivative having a fused ring is not limited, and an ordinary amino acid synthesis method is used. The production method is performed according to the method for producing a 4-borono-phenylalanine derivative described above; first, for example, a protected amino acid is reacted with an organic halide represented by the following general formula (V) in the presence of an organic solvent, a basic aqueous solution, and a phase transfer catalyst.

[Formula 13]

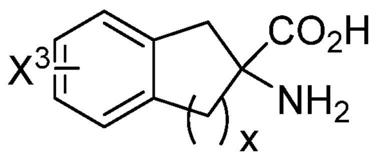

(V)

In the formula (V), x may be any integer of 1 to 3. $X^3$ represents halogen. An amino group or a hydroxyl group may be optionally protected. These can be obtained from commercially available products; for example, 2-amino-5-bromo-2,3-dihydro-1H-indene-2-carboxylic acid hydrochloride can be obtained from Enamine.

Other cyclic amino acids that are unavailable can be prepared by a Strecker reaction from indene or tetranone having a corresponding substituent. For example, one can be prepared by forming hydantoin with potassium cyanide and ammonium carbonate from a corresponding indene or tetranone, followed by hydrolysis with sodium hydroxide and subsequent decarboxylation with hydrochloric acid.

[L-type amino acid transporter 1 (LAT1)-selective agent]

The 4-borono-phenylalanine derivative of the present invention can be used for a LAT1-selective agent in the form of the above-described compound or pharmaceutically acceptable salt as received, or in the form of a pharmaceutical

preparation known to those skilled in the art in admixture with a pharmaceutically acceptable carrier, or in the form of encapsulation in micro/nanoparticles, or the like. Here, the LAT1-selective agent means that it is easily taken up by cells that express LAT1 and is hardly taken up by normal cells that express LAT2. LAT1 is abundantly expressed in cancer cells, and the effect of boron neutron capture therapy can be enhanced and damage to normal cells can be minimized if the compound has better selectivity of LAT1 compared to LAT2, which is expressed on the surface of normal cells. In the 4-borono-phenylalanine derivative of the present invention, the ratio of uptake into cells that express LAT1/uptake into cells that express LAT2 is preferably 2 times or more, more preferably 5 times or more, and still more preferably 10 times or more.

[Agent for boron neutron capture therapy (BNCT)]

The borono-phenylalanine derivative of the present invention can be conveniently used for BNCT in the form of the above-described compound or pharmaceutically acceptable salt as received, or in the form of a pharmaceutical preparation known to those skilled in the art in admixture with a pharmaceutically acceptable carrier, or in the form of encapsulation in micro/nanoparticles, or the like.

Treatment with a pharmaceutical preparation of the borono-phenylalanine derivative of the present invention is carried out by administration via any suitable administration route in such a way that the borono-phenylalanine derivative accumulates in a target tumor. The borono-phenylalanine derivative is preferably concentrated in the tumor before irradiation, and a tumor : blood ratio before irradiation is at least 1.5 or more : 1, and preferably 2 or more : 1. The borono-phenylalanine derivative may be administered at a time or continuously. In some cases, the borono-phenylalanine derivative may be administered separately. After the compound desirably accumulates in the tumor, the site is irradiated with an effective amount of low-energy neutron rays (for example, epithermal neutron rays). The site can be irradiated through the skin, or the site can be fully or partially exposed prior to irradiation. Administration of the borono-phenylalanine derivative and the subsequent irradiation may be repeated, as necessary. If desired, in order to reduce the tumor to a surgically possible extent, treatment with a borono-phenylalanine derivative may be performed and followed by a surgical procedure. Alternatively, after performing a surgical procedure, the remaining tumor is destroyed using a borono-phenylalanine derivative of the present invention. In another aspect, an appropriate amount of a borono-phenylalanine derivative is administered to a patient, followed by irradiation with an effective amount of 252 californium, which is a naturally occurring neutron radiant. Preferably, this is inserted into the tumor and then removed within a proper time.

Here, the type of tumor is not particularly limited, and brain tumors including glioblastoma and malignant glioma, head and neck cancers, malignant melanoma, breast cancer, prostate cancer, and the like can be particularly suitably targeted. In addition, epithelial cell cancers such as lung cancer, uterine cancer, kidney cancer, and liver cancer, various sarcomas, and the like can also be targeted.

The borono-phenylalanine derivative of the present invention may be administered orally and parenterally. In the case of parenteral administration, the borono-phenylalanine derivative may be administered intraarterially (for example, via carotid artery), intramuscularly, subcutaneously, intramedullary, intrathecally, intraventricularly, intravenously, intraperitoneally, or intranasally.

The pharmaceutical preparation may be formulated in any form such as powders, granules, fine granules, dry syrups, tablets, capsules, injections, and liquids and solutions. Depending on the dosage form, the pharmaceutical preparation may be administered to patients by a pharmaceutically known procedure in admixture with suitable additives and/or pharmaceutically acceptable carriers, alone, or in combination with other agents. Examples of the additives include excipients; disintegrators; binders; lubricants; diluents; buffers such as phosphoric acid, citric acid, succinic acid, acetic acid, other organic acids, and salts thereof; isotonizing agents; antiseptics; humectants; emulsifiers; dispersing agents; stabilizers; solubilizers; antioxidants such as ascorbic acid; low molecular weight (less than about 10 residues) polypeptides (for example, polyarginine or tripeptides); proteins (for example, serum albumin, gelatin, or immunoglobulin); hydrophilic polymers (for example, polyvinylpyrrolidone); amino acids (for example, glycine, glutamic acid, aspartic acid, or arginine); monosaccharides, disaccharides, and other carbohydrates (including cellulose or derivatives thereof, glucose, mannose, or dextrin); chelating agents (for example, EDTA); sugar alcohols (for example, mannitol or sorbitol); counter ions (for example, sodium); and/or nonionic surfactants (for example, polysorbate, poloxamer) . The pharmaceutical preparation can be prepared by appropriately mixing with, or diluting and dissolving together with such pharmaceutical additives Carriers which can be preferably used are, but are not limited to, pharmaceutically inert aqueous carriers. Examples of such carriers include physiological saline, buffered physiological saline, dextrose, and water. In one embodiment of the present invention, the pharmaceutically acceptable carriers are pharmaceutically inactive. Suitable additives and/or pharmaceutically acceptable carriers are non-toxic to recipients at the dosages and concentrations employed. Particularly preferred for the pharmaceutical preparation are injections prepared with an aqueous carrier.

Techniques for formulation and administration are described, for example, in the latest edition of the Japanese Pharmacopoeia and its latest supplement, and in the final version of "REMINGTON'S PHARMACEUTICAL SCIENCES" (Maack Publishing Co., Easton, PA).

A pharmaceutical preparation of the borono-phenylalanine derivative of the present invention is an agent contained in an amount effective for the objective agent to achieve the intended purpose, and the "therapeutically effective amount" or "pharmacologically effective amount" is well recognized by a person ordinarily skilled in the art, and refers to an amount of an agent effective to produce a pharmacological result. Determination of a therapeutically effective dose is well known to a person ordinarily skilled in the art.

The therapeutically effective amount as referred to herein means an amount of the agent to alleviate the disease state by irradiation after the administration. The therapeutic effect and toxicity of such compounds can be determined by standard pharmaceutical procedures in cell cultures or experimental animals. The dose is preferably in the range of circulating concentrations that include the $ED_{50}$ with little or no toxicity. This dose varies within this range depending on the administration form used, the sensitivity of patients, and the administration route. As an example, the dose is appropriately chosen depending on the age or other conditions of patients, the kind of diseases, the kind of composites to be used, etc. Preferred doses can be, but are not limited to, 5 to 1000 mg/kg administered in one treatment. In particular, the dose of the derivative may be 5 to 500 mg, and more preferably 6 to 480 mg, per 1 kg of the weight of the subject to be treated.

[Diagnostic agent containing radioisotope]

The borono-phenylalanine derivatives of the present invention can also be prepared as agents containing radioisotopes. In the case of preparation as an agent containing a radioisotope, typically, but not limitingly, $^{18}F$ can be used as an F atom contained in the compound, $^{131}I$ or $^{123}I$ can be used as an I atom contained in the compound, or $^{11}C$ can be used as a C atom contained in the compound. These radioisotopes may be typically introduced into $R^1$ to $R^4$. Thus-obtained compounds can be used for, for example, RI inspection or nuclear medicine inspection. These include, but are not limited to, agents for scintigraphy tomography, single photon emission computed tomography (SPECT), and positron emission tomography (PET). That is, the 4-borono-phenylalanine derivative of the present invention containing radioactivity is administered to a subject as a PET agent or an SPECT agent, and an image is acquired before treatment, and thus information such as the bioaccumulation distribution of the derivative and the tumor tissue/normal tissue abundance ratio (T/N ratio) can be obtained. Based on these pieces of information, it is also possible to presume the therapeutic effect of BNCT and formulate a study or treatment plan. In addition, the administration mode and the like are in accordance with the contents described in the section of [Agent for boron neutron capture therapy (BNCT)].

Examples

The present invention will be described in more detail with reference to the following examples, but the present invention is not limited thereto.

In the following examples, compounds were analyzed, and separated and purified using the following instruments and reagents.

·NMR spectrum:
(JEOL RESONANCE/JNM-ECZ500R/500MHz)
·LC/MS data:
(Waters XEVO G2-XS QTof/UPLC)
What is shown in the LC/MS spectrum of the compound of the present invention was measured by the following method, and was represented by a retention time (unit: minute) and $[M+H]^+$ or $[M+Na]^+$.
Column: Waters ACQUITY UPLC BEH C18 1.7$\mu$m, 2.1 × 50 mm column
Flow rate: 0.8 mL/min
Mobile phase: A; 0.1% aqueous formic acid solution, B; acetonitrile
Gradient: 5% (B, 0 min) ,100% (B, 3.50 min, linear gradient),100% (B, 6.00 min),5% (B, 7.00 min, linear gradient)
MS tune: Capilary (kV); 3.00, Sampling Cone; 40, Source Offset; 80, temperature Source; 150, Desolvation; 20, Cone Gas (L/h); 50, Desolvasion Gas (L/h); 1200.

(Example 1)

Synthesis of (S)-2-amino-3-(4-borono-2-fluorophenyl)-2-methylpropanoic acid

[Formula 14]

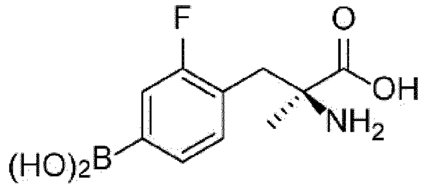

Step 1

Synthesis of tert-butyl (S)-2-amino-3-(4-bromo-2-fluorophenyl)-2-methylpropanoate

To a solution of tert-butyl 2-((4-chlorobenzylidene)amino)propanoate (7.16 g, 26.7 mmol), 4-bromo-1-(bromomethyl)-2-fluorobenzene (8.64 g, 32.3 mmol), and (R)-4,4-dibutyl-2,6-bis(3,4,5-trifluorophenyl)-4,5-dihydro-3H-dinaphtho[2,1-c:1',2'-e]azepinium bromide (19.9 mg, 26.6 μmol) in toluene (56 mL) was added dropwise an 80% aqueous cesium hydroxide solution (24. 9 g) at 5°C or lower, and then the mixture was stirred at -5°C for 42 hours. Water (30 mL) was added to the reaction mixture, and the mixture was extracted twice with toluene (50 mL), and then the organic phase was washed with saturated saline (50 mL). The organic phase was dried over anhydrous sodium sulfate and then concentrated under reduced pressure, affording a concentrate. The concentrate was used for the next step as received without purification.

The concentrate obtained was dissolved in tetrahydrofuran (50 mL), a 25% aqueous citric acid solution (203 g) was added thereto, and the mixture was stirred at room temperature for 3 hours. After completion of the reaction, the residue obtained by distilling off tetrahydrofuran under reduced pressure was washed with ethyl acetate (100 mL), and then the aqueous phase was adjusted to pH 8 or larger with potassium carbonate. The aqueous phase was extracted twice with ethyl acetate (100 mL), and then the organic phase was washed with saturated saline (100 mL). The organic phase was dried over anhydrous sodium sulfate and then concentrated under reduced pressure, and the resulting residue was purified by silica gel column chromatography (n-hexane : ethyl acetate = 1 : 1 (v/v)), affording tert-butyl (S)-2-amino-3-(4-bromo-2-fluorophenyl)-2-methylpropanoate (6.17 g, yield 70%).

$^1$H NMR ($CDCl_3$); 1.44 (s, 9H, t-Bu), 2.80 (dd, J = 9.5, 14.0 Hz, 1H, β-H), 3.15 (dd, J = 6.0, 14.0 Hz, 1H, β-H), 3.66 (dd, J = 5.5, 9.0 Hz, 1H, α-H), 7.12 (d, J = 8.0 Hz, 1H, ArH), 7.42 (dd, J = 1.5, 8.0 Hz, 1H, ArH), 7.99 (d, J = 2.0 Hz, 1H, ArH) .

Step 2

Synthesis of tert-butyl

(S)-3-(4-bromo-2-fluorophenyl)-2-((tert-butoxycarbonyl)amin o)-2-methylpropanoate

tert-Butyl (S)-2-amino-3-(4-bromo-2-fluorophenyl)-2-methylpropanoate (5.00 g, 15.1 mmol) obtained in Step 1 was dissolved in acetonitrile (50 mL). Sodium carbonate (3.20 g, 30.2 mmol) in water (50 mL), $Boc_2O$ (3.93 g, 18.0 mmol) were added to the solution, and then the mixture was stirred at room temperature overnight. After the reaction, an aqueous phase obtained by concentrating acetonitrile under reduced pressure was extracted with ethyl acetate (50 mL), and the organic phase was dried over anhydrous magnesium sulfate, and then the solvent was concentrated under reduced pressure. The resulting residue was powdered with n-hexane, collected by filtration, and washed with n-hexane, affording tert-butyl (S)-3-(4-bromo-2-fluorophenyl)-2-((tert-butoxycarbonyl)amin o)-2-methylpropanoate (4.90 g, yield 75%).

$^1$H NMR ($CDCl_3$); 1.46 (s, 21H, t-Bu×2+α-Me), 3.26-3.34 (m, 2H, β-H), 5.11 (brs, 1H, amide), 7. 04 (t, J = 8.0 Hz, 1H, ArH), 7.17-7.20 (m, 2H, ArH).

Step 3

Production of

(S)-(4-(3-(tert-butoxy)-2-((tert-butoxycarbonyl)amino)-2-me thyl-3-oxopropyl)-3-fluorophenyl)boronic acid

$Pd(dba)_2$ (325 mg, 0.565 mmol) and tricyclohexylphosphine (222 mg, 0.791 mmol) were added to DMSO (49

mL), and the mixture was stirred at room temperature for 30 minutes.

Bis(pinacolato) diboron (3.44 g, 13. 6 mmol), potassium acetate (1.66 g, 17.0 mmol), and tert-butyl (S)-3-(4-bromo-2-fluorophenyl)-2-((tert-butoxycarbonyl)amin o)-2-methylpropanoate (4.90 g, 11.3 mmol) were added thereto, and the mixture was stirred at 80°C for 18 hours. The reaction mixture was cooled, and then ethyl acetate (49 mL) and distilled water (25 mL) were added thereto. After stirring for about 5 minutes, Celite filtration was conducted and the resulting filtrate was transferred to a separatory funnel. The aqueous phase was further extracted with ethyl acetate (25 mL), and the resulting organic phases were combined, washed with saturated saline (49 mL), dried over anhydrous magnesium sulfate, and then distilled off under reduced pressure, and the residue thus obtained was subjected to silica gel column chromatography, affording an intermediate.

An acetone solution (245 mL) of the obtained intermediate was added to a mixed liquid of sodium periodate (6.04 g, 28.3 mmol) and ammonium acetate (2.18 g, 28.3 mmol) in water (245 mL) . The mixture was stirred at room temperature for 2 days. Acetone in the reaction liquid was distilled off under reduced pressure, and the resulting aqueous solution was extracted twice with ethyl acetate (123 mL) . The organic phase was dried over anhydrous magnesium sulfate and then distilled off under reduced pressure, and the resulting residue was purified by silica gel column chromatography, affording

(S)-(4-(3-(tert-butoxy)-2-((tert-butoxycarbonyl)amino)-2-me thyl-3-oxopropyl)-3-fluorophenyl)boronic acid (2.38 g, yield 53%) .

$^1$H NMR (DMSO-$d_6$); 1.08 (s, 3H, α-Me), 1.37 (s, 9H, t-Bu), 1.41 (s, 9H, t-Bu), 2.95 (d, J = 13.5 Hz, 1H, β-H), 3.27 (d, J = 13.5 Hz, 1H, β-H), 6.95 (brs, 1H, amide), 7.07 (t, J = 7.5 Hz, 1H, ArH), 7.44-7.50 (m, 2H, ArH), 8.17 (s, 2H, $B(OH)_2$).

Step 4

Production of (S)-2-amino-3-(4-borono-2-fluorophenyl)-2-methylpropanoic acid

(S)-(4-(3-(tert-Butoxy)-2-((tert-butoxycarbonyl)amino )-2-methyl-3-oxopropyl)-3-fluorophenyl)boronic acid (1.00 g, 2.52 mmol) was dissolved in trifluoroacetic acid (10 mL) and the solution was stirred for 3 hours. The reaction mixture was concentrated under reduced pressure, a small amount of distilled water was added thereto, and the mixture was neutralized with sodium carbonate. The resulting precipitate was collected by filtration and washed with cold water, affording

(S)-2-amino-3-(4-borono-2-fluorophenyl)-2-methylpropanoic acid (0.36 g, yield 59%).

$^1$H NMR (3.5% DCl in $D_2O$); 1.68 (s, 3H, α-Me), 3.34 (s, 2H, β-H), 7.35 (t, J = 7.5 Hz, 1H, ArH), 7.45-7.54 (m, 2H, ArH) .

(Example 2)

The following compound was synthesized in the same manner as in Example 1 except that (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 15]

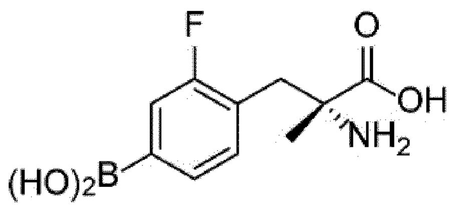

(R)-2-Amino-3-(4-borono-2-fluorophenyl)-2-methylpropa noic acid

$^1$H NMR (3.5% DCl in $D_2O$); 1.68 (s, 3H, α-Me), 3.34 (s, 2H, β-H), 7.35 (t, J = 7.5 Hz, 1H, ArH), 7.45-7.54 (m, 2H, ArH) .

(Example 3)

The following compound was synthesized in the same manner as in Example 1 except that 4-bromo-1-(bromomethyl)-2-fluorobenzene was changed to 4-bromo-1-(bromomethyl)-2-methylbenzene.

[Formula 16]

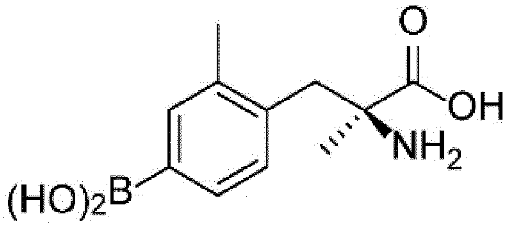

(S)-2-Amino-3-(4-borono-2-methylphenyl)-2-methylpropa noic acid

$^{1}H$ NMR (3.5% DCl in $D_2O$); 1.66 (s, 3H, α-Me), 2.34 (s, 3H, Ar-Me), 3.33-3.34 (m, 2H, β-H), 7.24 (d, J = 7.5 Hz, 2H, ArH), 7.56 (d, J = 7.5 Hz, 1H, ArH), 7.62 (s, 1H, ArH).

(Example 4)

The following compound was synthesized in the same manner as in Example 1 except that 4-bromo-1-(bromomethyl)-2-fluorobenzene was changed to 4-bromo-1-(bromomethyl)-2-methylbenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 17]

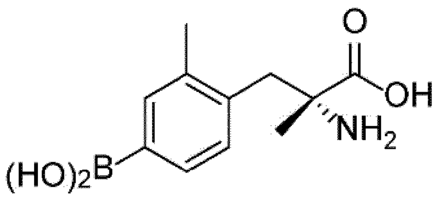

(R)-2-Amino-3-(4-borono-2-methylphenyl)-2-methylpropa noic acid

$^{1}H$ NMR (3.5% DCl in $D_2O$); 1.66 (s, 3H, α-Me), 2.34 (s, 3H, Ar-Me), 3.33-3.34 (m, 2H, β-H), 7.24 (d, J = 7.5 Hz, 2H, ArH), 7.56 (d, J = 7.5 Hz, 1H, ArH), 7.62 (s, 1H, ArH).

(Example 5)

The following compound was synthesized in the same manner as in Example 1 except that 4-bromo-1-(bromomethyl)-2-fluorobenzene was changed to 4-bromo-1-(bromomethyl)-2-(trifluoromethyl)benzene.

[Formula 18]

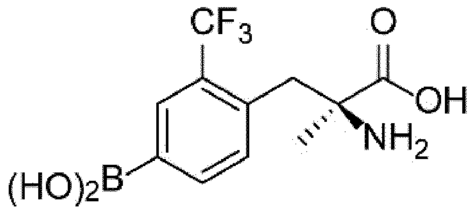

(S)-2-Amino-3-(4-borono-2-(trifluoromethyl)phenyl)-2-methylpropanoic acid

$^1H$ NMR (3.5% DCl in $D_2O$); 1.71 (s, 3H, α-Me), 3.50 (d, J = 4.0 Hz, 2H, β-H), 7.48 (d, J = 7.0 Hz, 2H, ArH), 7.92 (d, J = 7.5 Hz, 1H, ArH), 8.08 (s, 1H, ArH).

(Example 6)

The following compound was synthesized in the same manner as in Example 1 except that 4-bromo-1-(bromomethyl)-2-fluorobenzene was changed to 4-bromo-1-(bromomethyl)-2-(trifluoromethyl)benzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 19]

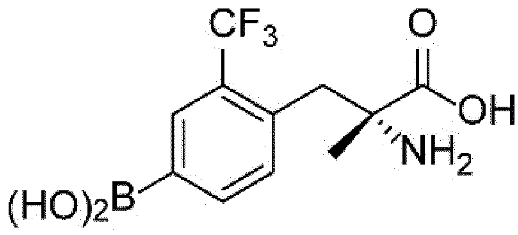

(R)-2-Amino-3-(4-borono-2-(trifluoromethyl)phenyl)-2-methylpropanoic acid

$^1H$ NMR (3.5% DCl in $D_2O$); 1.71 (s, 3H, α-Me), 3.50 (d, J = 4.0 Hz, 2H, β-H), 7.48 (d, J = 7.0 Hz, 2H, ArH), 7.92 (d, J = 7.5 Hz, 1H, ArH), 8.08 (s, 1H, ArH).

(Example 7)

The following compound was synthesized in the same manner as in Example 1 except that 4-bromo-1-(bromomethyl)-2-fluorobenzene was changed to 4-bromo-1-(bromomethyl)-2-methoxybenzene.

[Formula 20]

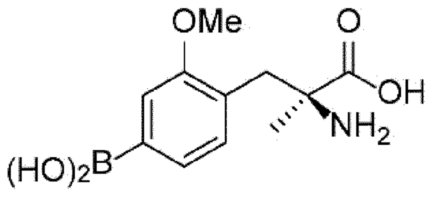

(S)-2-Amino-3-(4-borono-2-methoxyphenyl)-2-methylprop anoic acid

$^1H$ NMR (3.5% DCl in $D_2O$); 1.67 (s, 3H, α-Me), 3.28 (s, 2H, β-H), 3.85 (s, 3H, OMe), 7.26 (d, J = 7.5 Hz, 1H,

ArH), 7.34 (d, J = 8.0 Hz, 1H, ArH), 7.35 (s, 1H, ArH).

(Example 8)

The following compound was synthesized in the same manner as in Example 1 except that 4-bromo-1-(bromomethyl)-2-fluorobenzene was changed to 4-bromo-1-(bromomethyl)-2-methoxybenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 21]

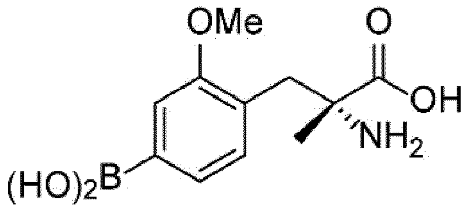

(R)-2-Amino-3-(4-borono-2-methoxyphenyl)-2-methylprop anoic acid

$^1H$ NMR (3.5% DCl in $D_2O$); 1.67 (s, 3H, α-Me), 3.28 (s, 2H, β-H), 3.85 (s, 3H, OMe), 7.26 (d, J= 7.5 Hz, 1H, ArH), 7.34 (d, J = 8.0 Hz, 1H, ArH), 7.35 (s, 1H, ArH).

(Example 9)

The following compound was synthesized in the same manner as in Example 1 except that

4-bromo-1-(bromomethyl)-2-fluorobenzene was changed to 4-bromo-1-(bromomethyl)-2-chlorobenzene.

[Formula 22]

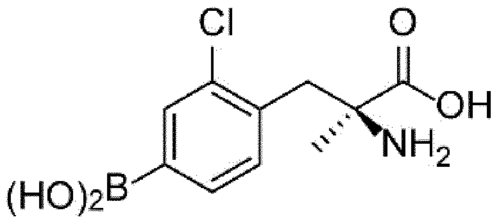

(S)-2-Amino-3-(4-borono-2-chlorophenyl)-2-methylpropa noic acid

$^1H$ NMR (3.5% DCl in $D_2O$); 1.67 (s, 3H, α-Me), 3.38 (d, J = 14.5 Hz, 1H, β-H), 3.48 (d, J = 14.0 Hz, 1H, β-H), 7.34 (d, J = 8.0 Hz, 1H, ArH), 7.61 (dd, J = 1.0, 7.5 Hz, 1H, ArH), 7.74 (d, J = 1.5 Hz, 1H, ArH).

(Example 10)

The following compound was synthesized in the same manner as in Example 1 except that 4-bromo-1-(bromomethyl)-2-fluorobenzene was changed to 4-bromo-1-(bromomethyl)-2-chlorobenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 23]

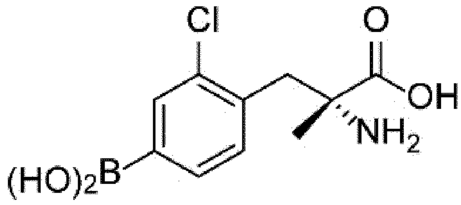

(R)-2-Amino-3-(4-borono-2-chlorophenyl)-2-methylpropa noic acid

$^1$H NMR (3.5% DCl in $D_2O$); 1.67 (s, 3H, α-Me), 3.38 (d, J = 14.5 Hz, 1H, β-H), 3.48 (d, J = 14.0 Hz, 1H, β-H), 7.34 (d, J = 8.0 Hz, 1H, ArH), 7.61 (dd, J = 1.0, 7.5 Hz, 1H, ArH), 7.74 (d, J = 1.5 Hz, 1H, ArH).

(Example 11)

The following compound was synthesized in the same manner as in Example 1 except that 4-bromo-1-(bromomethyl)-2-fluorobenzene was changed to 5-bromo-2-(bromomethyl)-1,3-difluorobenzene.

[Formula 24]

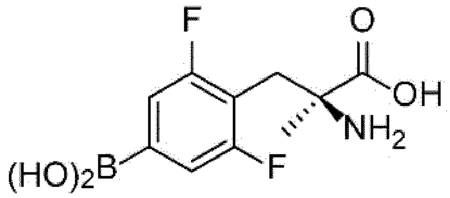

(S)-2-Amino-3-(4-borono-2,6-difluorophenyl)-2-methylp ropanoic acid

$^1$H NMR (3.5% DCl in $D_2O$); 1.65 (s, 3H, α-Me), 3.37 (s, 2H, β-H), 7.11 (d, J = 8.0 Hz, 2H, ArH).

(Example 12)

The following compound was synthesized in the same manner as in Example 1 except that 4-bromo-1-(bromomethyl)-2-fluorobenzene was changed to 5-bromo-2-(bromomethyl)-1,3-difluorobenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 25]

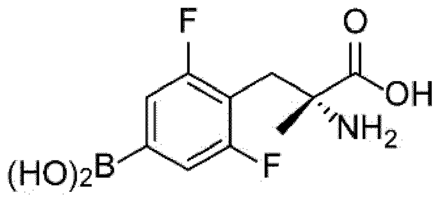

(R)-2-Amino-3-(4-borono-2,6-difluorophenyl)-2-methylp ropanoic acid

$^1$H NMR (3.5% DCl in $D_2O$); 1.65 (s, 3H, α-Me), 3.37 (s, 2H, β-H), 7.11 (d, J = 8.0 Hz, 2H, ArH).

(Example 13)

The following compound was synthesized in the same manner as in Example 1 except that 4-bromo-1-(bromomethyl)-2-fluorobenzene was changed to 1-bromo-4-(bromomethyl)-2-chloro-5-fluorobenzene.

[Formula 26]

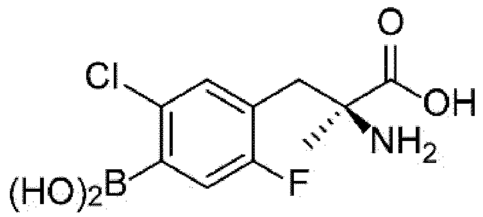

(S)-2-Amino-3-(4-borono-5-chloro-2-fluorophenyl)-2-me thylpropanoic acid

$^1$H NMR (3.5% DCl in $D_2O$); 1.66 (s, 3H, α-Me), 3.29 (s, 2H, β-H), 7.28 (dd, J = 2.0, 10.0 Hz, 1H, ArH), 7.34 (d, J = 5.0 Hz, 1H, ArH).

(Example 14)

The following compound was synthesized in the same manner as in Example 1 except that 4-bromo-1-(bromomethyl)-2-fluorobenzene was changed to 1-bromo-4-(bromomethyl)-2,3-difluorobenzene.

[Formula 27]

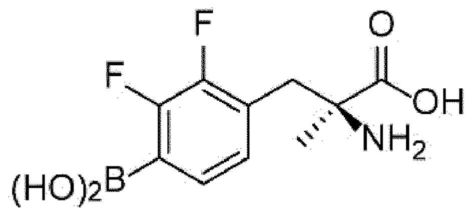

(S)-2-Amino-3-(4-borono-2,3-difluorophenyl)-2-methylp ropanoic acid

$^1$H NMR (3.5% DCl in $D_2O$); 1.67 (s, 3H, α-Me), 3.36 (s, 2H, β-H), 7.11 (t, J = 7.0 Hz, 1H, ArH), 7.36 (t, J = 7.0 Hz, 1H, ArH).

(Example 15)

The following compound was synthesized in the same manner as in Example 1 except that 4-bromo-1-(bromomethyl)-2-fluorobenzene was changed to 1-bromo-4-(bromomethyl)-2-fluorobenzene.

[Formula 28]

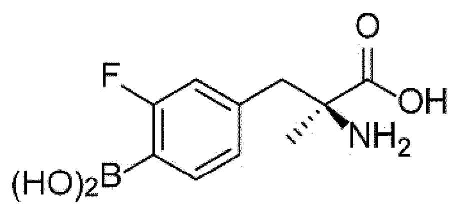

(S)-2-Amino-3-(4-borono-3-fluorophenyl)-2-methylpropa noic acid

$^1$H NMR (3.5% DCl in $D_2O$); 1.76 (s, 3H, α-Me), 3.23 (d, J = 14.0 Hz, 1H, β-H), 3.23 (d, J = 14.0 Hz, 1H, β-H), 7.05 (d, J = 9.5 Hz, 1H, ArH), 7.16 (dd, J = 1.0, 8.0 Hz, 1H, ArH), 7.68 (t, J = 7.0 Hz, 1H, ArH).

(Example 16)

The following compound was synthesized in the same manner as in Example 1 except that 4-bromo-1-(bromomethyl)-2-fluorobenzene was changed to 1-bromo-4-(bromomethyl)-2-fluorobenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 29]

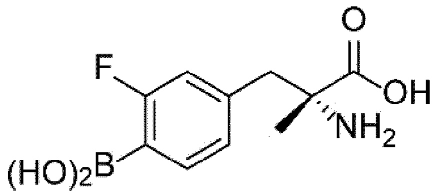

(R)-2-Amino-3-(4-borono-3-fluorophenyl)-2-methylpropa noic acid

$^{1}H$ NMR (3.5% DCl in $D_2O$); 1.76 (s, 3H, α-Me), 3.23 (d, J = 14.0 Hz, 1H, β-H), 3.23 (d, J = 14.0 Hz, 1H, β-H), 7.05 (d, J = 9.5 Hz, 1H, ArH), 7.16 (dd, J = 1.0, 8.0 Hz, 1H, ArH), 7.68 (t, J = 7.0 Hz, 1H, ArH).

(Example 17)

The following compound was synthesized in the same manner as in Example 1 except that 4-bromo-1-(bromomethyl)-2-fluorobenzene was changed to 1-bromo-4-(bromomethyl)-2-methylbenzene.

[Formula 30]

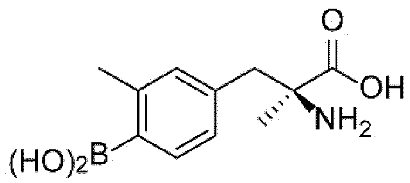

(S)-2-Amino-3-(4-borono-3-methylphenyl)-2-methylpropa noic acid

$^{1}H$ NMR (3.5% DCl in $D_2O$); 1.69 (s, 3H, α-Me), 2.45 (s, 3H, Me), 3.08 (d, J = 14.0 Hz, 1H, β-H), 3.32 (d, J = 14.0 Hz, 1H, β-H), 7.08-7.10 (m, 2H, ArH), 7.49 (d, J = 6. 0 Hz, 1H, ArH) .

(Example 18)

The following compound was synthesized in the same manner as in Example 1 except that 4-bromo-1-(bromomethyl)-2-fluorobenzene was changed to 1-bromo-4-(bromomethyl)-2-methylbenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 31]

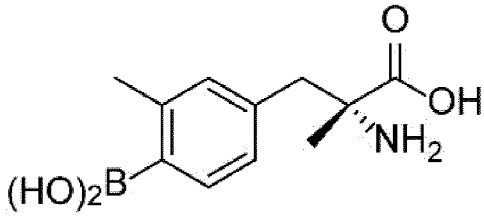

(R)-2-Amino-3-(4-borono-3-methylphenyl)-2-methylpropa noic acid

$^1$H NMR (3.5% DCl in $D_2O$); 1.69 (s, 3H, α-Me), 2.45 (s, 3H, Me), 3.08 (d, J = 14.0 Hz, 1H, β-H), 3.32 (d, J = 14.0 Hz, 1H, β-H), 7.08-7.10 (m, 2H, ArH), 7.49 (d, J = 6. 0 Hz, 1H, ArH) .

(Example 19)

The following compound was synthesized in the same manner as in Example 1 except that tert-butyl 2-((4-chlorobenzylidene)amino)propanoate was changed to tert-butyl 2-((4-chlorobenzylidene)amino)butanoate, and 4-bromo-1-(bromomethyl)-2-fluorobenzene was changed to 4-iodobenzyl bromide.

[Formula 32]

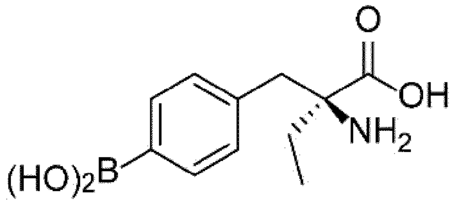

(S)-2-Amino-2-(4-boronobenzyl)butanoic acid

$^1$H NMR (3.5% DCl in $D_2O$); 1.04 (t, J = 7.5 Hz, 3H, α-$CH_2CH_3$), 1.97-2.01 (m, 1H, α-$CH_2CH_3$), 2.15-2.19 (m, 1H, α-$CH_2CH_3$), 3.17 (d, J = 14.0 Hz, 1H, β-H), 3.42 (d, J = 14.0 Hz, 1H, β-H), 7.32 (d, J = 8.0 Hz, 1H, ArH), 7.76 (d, J = 8.0 Hz, 1H, ArH).

(Example 20)

The following compound was synthesized in the same manner as in Example 1 except that tert-butyl 2-((4-chlorobenzylidene)amino)propanoate was changed to tert-butyl 2-((4-chlorobenzylidene)amino)butanoate, 4-bromo-1-(bromomethyl)-2-fluorobenzene was changed to 4-iodobenzyl bromide, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 33]

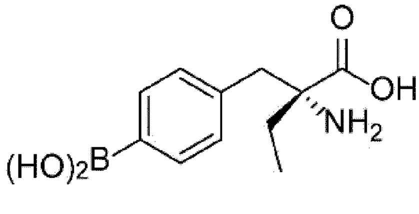

(R)-2-Amino-2-(4-boronobenzyl)butanoic acid

$^1$H NMR (3.5% DCl in $D_2O$); 1.04 (t, J = 7.5 Hz, 3H, α-$CH_2CH_3$), 1.97-2.01 (m, 1H, α-$CH_2CH_3$), 2.15-2.19 (m, 1H, α-$CH_2CH_3$), 3.17 (d, J = 14.0 Hz, 1H, β-H), 3.42 (d, J = 14.0 Hz, 1H, β-H), 7.32 (d, J = 8.0 Hz, 1H, ArH), 7.76 (d, J = 8.0 Hz, 1H, ArH).

(Example 21)

The following compound was synthesized in the same manner as in Example 1 except that 4-bromo-1-(bromomethyl)-2-fluorobenzene was changed to 5-bromo-2-(bromomethyl)benzonitrile.

[Formula 34]

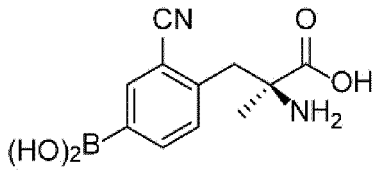

(S)-2-Amino-3-(4-borono-2-cyanophenyl)-2-methylpropan oic acid

$^1$H NMR (3.5% DCl in $D_2O$); 8.26 (d, J = 1.5 Hz, 1H, ArH), 8.03 (dd, J = 1.5, 7.5 Hz, 1H, ArH), 7.49 (d, J = 7.5 Hz, 1H, ArH), 3.50 (d, J = 16.5 Hz, 1H, $CH_2$), 3.30 (d, J = 16.5 Hz, 1H, $CH_2$), 2. 09 (s, 3H, $CH_3$), 1.71 (s, 3H, $CH_3$).

(Example 22)

The following compound was synthesized in the same manner as in Example 1 except that 4-bromo-1-(bromomethyl)-2-fluorobenzene was changed to 1-bromo-4-(bromomethyl)-2-chloro-5-fluorobenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 35]

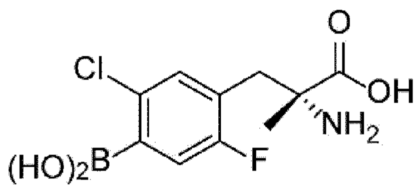

(R)-2-Amino-3-(4-borono-5-chloro-2-fluorophenyl)-2-me thylpropanoic acid

$^1$H NMR (3.5% DCl in $D_2O$); 1.66 (s, 3H, α-Me), 3.29 (s, 2H, β-H), 7.28 (dd, J = 2.0, 10.0 Hz, 1H, ArH), 7.34 (d, J = 5.0 Hz, 1H, ArH).

(Example 23)

The following compound was synthesized in the same manner as in Example 1 except that 4-bromo-1-(bromomethyl)-2-fluorobenzene was changed to 1-bromo-4-(bromomethyl)-5-chloro-2-fluorobenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 36]

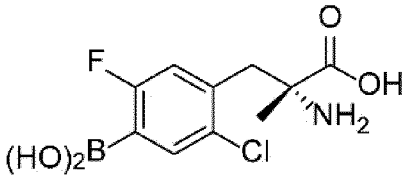

(R)-2-Amino-3-(4-borono-2-chloro-5-fluorophenyl)-2-me thylpropanoic acid

$^1H$ NMR (TFA in $D_2O$); 7.65 (dd, J = 3.5, 6.0 Hz, 1H, ArH), 7.10 (dd, J = 2.5, 9.5 Hz, 1H, ArH), 3.47 (d, J = 15.0 Hz, 1H, $CH_2$), 3.38 (d, J = 15.0 Hz, 1H, $CH_2$), 1.68 (s, 3H, $CH_3$).

(Example 24)

The following compound was synthesized in the same manner as in Example 1 except that 4-bromo-1-(bromomethyl)-2-fluorobenzene was changed to 1-bromo-4-(bromomethyl)-2,3-difluorobenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 37]

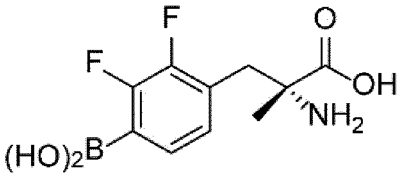

(R)-2-Amino-3-(4-borono-2,3-difluorophenyl)-2-methylp ropanoic acid

$^1H$ NMR (TFA in $D_2O$); 1.67 (s, 3H, $\alpha$-Me), 3.36 (s, 2H, $\beta$-H), 7.11 (t, J = 7.0 Hz, 1H, ArH), 7.36 (t, J = 7.0 Hz, 1H, ArH).

(Example 25)

The following compound was synthesized in the same manner as in Example 1 except that 4-bromo-1-(bromomethyl)-2-fluorobenzene was changed to 4-bromo-1-(bromomethyl)-2-nitrobenzene.

[Formula 38]

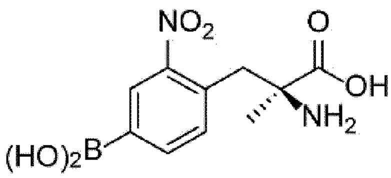

(S)-2-Amino-3-(4-borono-2-nitrophenyl)-2-methylpropan oic acid

$^1H$ NMR (TFA in $D_2O$); 8.33 (d, J = 1.5 Hz, 1H, ArH), 7.98 (d, J = 8.0 Hz, 1H, ArH), 7.50 (dd, J = 1.5, 8.0 Hz, 1H, ArH), 3.76 (d, J = 14.0 Hz, 1H, $CH_2$), 3.53 (d, J = 14.0 Hz, 1H, $CH_2$), 1.67 (s, 3H, $CH_3$).

(Example 26)

The following compound was synthesized in the same manner as in Example 1 except that 4-bromo-1-(bromomethyl)-2-fluorobenzene was changed to 4-(bromomethyl)-1-iodo-2-nitrobenzene.

[Formula 39]

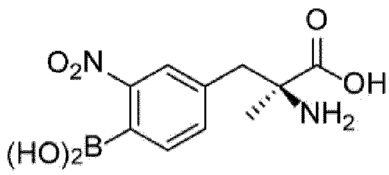

(S)-2-Amino-3-(4-borono-3-nitrophenyl)-2-methylpropan oic acid

$^1H$ NMR (TFA in $D_2O$); 8.14 (s, 1H, ArH), 7.68 (d, J = 1.5, 7.5 Hz, 1H, ArH), 7.63 (dd, J = 1.5, 7.5 Hz, 1H, ArH), 3.50 (d, J = 14.5 Hz, 1H, $CH_2$), 3.29 (d, J = 14.5 Hz, 1H, $CH_2$), 1.68 (s, 3H, $CH_3$).

(Example 27)

The following compound was synthesized in the same manner as in Example 1 except that 4-bromo-1-(bromomethyl)-2-fluorobenzene was changed to 4-(bromomethyl)-1-iodo-2-nitrobenzene.

[Formula 40]

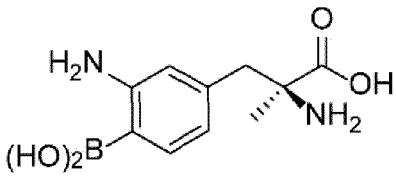

(S)-2-Amino-3-(3-amino-4-boronophenyl)-2-methylpropan oic acid

$^1H$ NMR (3.5% DCl in $D_2O$); 1.74 (s, 3H, $\alpha$-Me), 3.28 (d, J = 14.0 Hz, 1H, $\beta$-H), 3.48 (d, J = 14.5 Hz, 1H, $\beta$-H), 7.36 (s, 1H, ArH), 7.45 (d, J = 8.0 Hz, 1H, ArH), 7.93 (d, J = 7.5 Hz, 1H, ArH) .

(Example 28)

The following compound was synthesized in the same manner as in Example 1 except that 4-bromo-1-(bromomethyl)-2-fluorobenzene was changed to 4-bromo-1-(bromomethyl)-2-nitrobenzene.

[Formula 41]

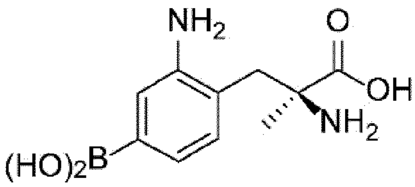

(S)-2-Amino-3-(2-amino-4-boronophenyl)-2-methylpropan oic acid

$^1H$ NMR (3.5% DCl in $D_2O$); 1.47 (s, 3H, $\alpha$-Me), 3.22 (d, J = 15.5 Hz, 1H, $\beta$-H), 3.43 (d, J = 15.5 Hz, 1H, $\beta$-H),

7.27 (s, 1H, ArH), 7.33 (d, J = 7.5 Hz, 1H, ArH), 7.47 (d, J = 7.5 Hz, 1H, ArH).

(Example 29)

The following compound was synthesized in the same manner as in Example 1 except that 4-bromo-1-(bromomethyl)-2-fluorobenzene was changed to 1-bromo-4-(bromomethyl)-5-chloro-2-fluorobenzene.

[Formula 42]

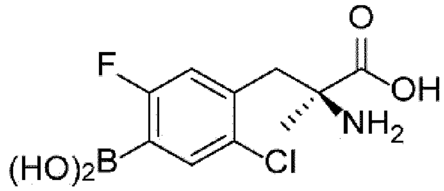

(S)-2-Amino-3-(4-borono-2-chloro-5-fluorophenyl)-2-me thylpropanoic acid

$^1$H NMR (TFA in $D_2O$); 7.65 (dd, J = 3.5, 6.0 Hz, 1H, ArH), 7.10 (dd, J = 2.5, 9.5 Hz, 1H, ArH), 3.47 (d, J = 15.0 Hz, 1H, $CH_2$), 3.38 (d, J = 15.0 Hz, 1H, $CH_2$), 1.68 (s, 3H, $CH_3$).

(Example 30)

The following compound was synthesized in the same manner as in Example 1 except that 4-bromo-1-(bromomethyl)-2-fluorobenzene was changed to 2-(benzyloxy)-4-bromo-1-(bromomethyl)benzene.

[Formula 43]

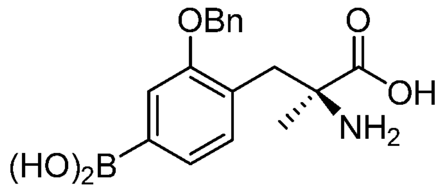

(S)-2-Amino-3-(2-(benzyloxy)-4-boronophenyl)-2-methyl propanoic acid

$^1$H NMR (3.5% DCl in $D_2O$); 1.77 (s, 3H, $\alpha$-Me), 3.33 (d, J = 14.5 Hz, 1H, $\beta$-H), 3.45 (d, J = 14.0 Hz, 1H, $\beta$-H), 5.10 (d, J = 11.5 Hz, 1H, $ArCH_2$), 5.16 (d, J = 12.0 Hz, 1H, $ArCH_2$), 7.39-7.52 (m, 6H, ArH), 7.62-7.63 (m, 2H, ArH).

(Example 31)

The following compound was synthesized in the same manner as in Example 1 except that 4-bromo-1-(bromomethyl)-2-fluorobenzene was changed to 2-(benzyloxy)-4-bromo-1-(bromomethyl)benzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 44]

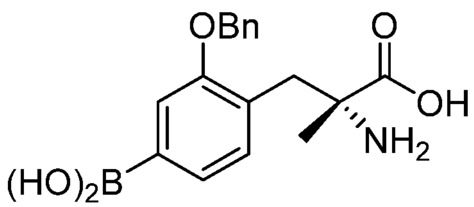

(R)-2-Amino-3-(2-(benzyloxy)-4-boronophenyl)-2-methyl propanoic acid

$^1$H NMR (3.5% DCl in $D_2O$); 1.77 (s, 3H, α-Me), 3.32 (d, J = 14.0 Hz, 1H, β-H), 3.44 (d, J = 13.5 Hz, 1H, β-H), 5.10 (d, J = 12.0 Hz, 1H, $ArCH_2$), 5.16 (d, J = 11.5 Hz, 1H, $ArCH_2$), 7.39-7.52 (m, 6H, ArH), 7.60-7.62 (m, 2H, ArH).

(Example 32)

The following compound was synthesized in the same manner as in Example 1 except that 4-bromo-1-(bromomethyl)-2-fluorobenzene was changed to 1-bromo-4-(bromomethyl)-2-methoxybenzene.

[Formula 45]

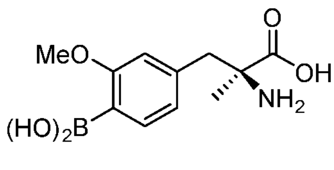

(S)-2-Amino-3-(4-borono-3-methoxyphenyl)-2-methylprop anoic acid

$^1$H NMR (3.5% DCl in $D_2O$); 1.74 (s, 3H, α-Me), 3.14 (d, J = 14.0 Hz, 1H, β-H), 3.38 (d, J = 14.0 Hz, 1H, β-H), 3.86 (s, 3H, $OCH_3$), 6.87 (d, J = 2.0 Hz, 1H, ArH), 6.93 (d, J = 7.5 Hz, 1H, ArH), 7.00 (dd, J = 2.5, 8.0 Hz, 1H, ArH).

(Example 33)

The following compound was synthesized in the same manner as in Example 1 except that 4-bromo-1-(bromomethyl)-2-fluorobenzene was changed to 1-bromo-4-(bromomethyl)-2-methoxybenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 46]

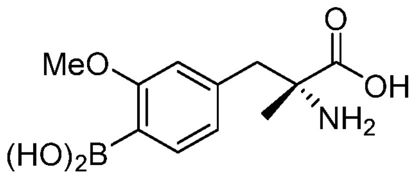

(R)-2-Amino-3-(4-borono-3-methoxyphenyl)-2-methylprop anoic acid

$^1$H NMR (3.5% DCl in $D_2O$); 1.72 (s, 3H, α-Me), 3.11 (d, J = 14.5 Hz, 1H, β-H), 3.36 (d, J = 14.0 Hz, 1H, β-H), 3.84 (s, 3H, $OCH_3$), 6.85 (s, 1H ArH), 6.91 (d, J = 8.5 Hz, 1H, ArH), 7.00 (dd, J = 2.0, 8.5 Hz, 1H, ArH).

(Example 34)

The following compound was synthesized in the same manner as in Example 1 except that 4-bromo-1-(bromomethyl)-2-fluorobenzene was changed to 1-bromo-4-(bromomethyl)-3-methylthiobenzene.

[Formula 47]

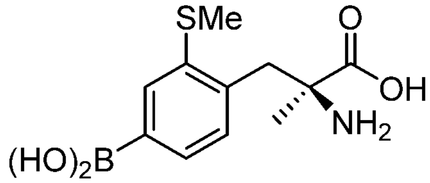

(S)-2-Amino-3-(4-borono-2-(methylthio)phenyl)-2-methy lpropanoic acid

$^1H$ NMR (3.5% DCl in $D_2O$); 1.39 (s, 3H, $\alpha$-Me), 2.18 (s, 3H, $SCH_3$), 3.04 (d, J = 15.0 Hz, 1H, $\beta$-H), 3.14 (d, J = 15.0 Hz, 1H, $\beta$-H), 6.92 (d, J = 7.5 Hz, 1H, ArH), 7.26 (d, J = 7.5 Hz, 1H, ArH), 7.40 (s, 1H, ArH).

(Example 35)

The following compound was synthesized in the same manner as in Example 1 except that 4-bromo-1-(bromomethyl)-2-fluorobenzene was changed to 4-bromo-1-(bromomethyl)-2-(difluoromethyl)benzene.

[Formula 48]

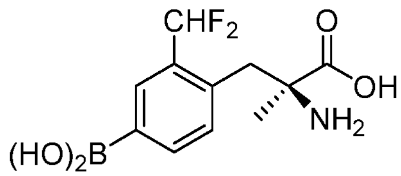

(S)-2-Amino-3-(4-borono-2-(difluoromethyl)phenyl)-2-m ethylpropanoic acid

$^1H$ NMR (3.5% DCl in $D_2O$); 1.82 (s, 3H, $\alpha$-Me), 3.54-3.55 (m, 2H, $\beta$-H), 6.92-7.14 (m, 1H, $CHF_2$). 7.47 (d, J = 8.0 Hz, 1H, ArH), 7.96 (d, J = 7.5 Hz, 1H, ArH), 8.05 (s, 1H, ArH).

(Example 36)

The following compound was synthesized in the same manner as in Example 1 except that 4-bromo-1-(bromomethyl)-2-fluorobenzene was changed to 4-bromo-1-(bromomethyl)-2-(difluoromethyl)benzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 49]

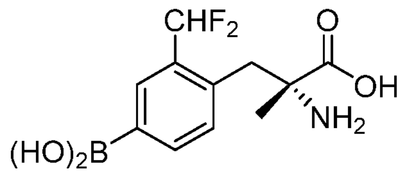

(R)-2-Amino-3-(4-borono-2-(difluoromethyl)phenyl)-2-m ethylpropanoic acid

$^1H$ NMR (3.5% DCl in $D_2O$); 1.77 (s, 3H, $\alpha$-Me), 3.49-3.50 (m, 2H, $\beta$-H), 6.92-7.13 (m, 1H, $CHF_2$), 7.41 (d, J = 8.0 Hz, 1H, ArH), 7.89 (d, J = 7.5 Hz, 1H, ArH), 7.99 (s, 1H, ArH).

(Example 37)

The following compound was synthesized in the same manner as in Example 1 except that 4-bromo-1-(bromomethyl)-2-fluorobenzene was changed to 1-bromo-4-(bromomethyl)-2-chlorobenzene.

[Formula 50]

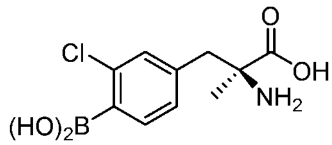

(S)-2-Amino-3-(4-borono-3-chlorophenyl)-2-methylpropa noic acid

$^1H$ NMR (3.5% DCl in $D_2O$); 1.68 (s, 3H, α-Me), 3.13 (d, J = 14.0 Hz, 1H, β-H), 3.35 (d, J = 14.5 Hz, 1H, β-H), 7.20 (dd, J = 1.0, 7.5 Hz, 1H, ArH), 7.30 (d, J = 1.0 Hz, 1H, ArH), 7.52 (d, J = 7.5 Hz, 1H, ArH).

(Example 38)

The following compound was synthesized in the same manner as in Example 1 except that 4-bromo-1-(bromomethyl)-2-fluorobenzene was changed to 1-bromo-4-(bromomethyl)-2-chlorobenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 51]

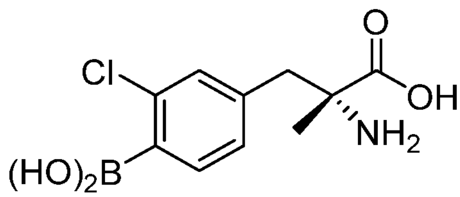

(R)-2-Amino-3-(4-borono-3-chlorophenyl)-2-methylpropa noic acid

$^1H$ NMR (3.5% DCl in $D_2O$); 1.69 (s, 3H, α-Me), 3.14 (d, J = 14.0 Hz, 1H, β-H), 3.36 (d, J = 14.0 Hz, 1H, β-H), 7.21 (d, J = 8.0 Hz, 1H, ArH), 7.30 (s, 1H, ArH), 7.53 (d, J = 7.5 Hz, 1H, ArH) .

(Example 39)

The following compound was synthesized in the same manner as in Example 1 except that 4-bromo-1-(bromomethyl)-2-fluorobenzene was changed to 1-bromo-4-(bromomethyl)-2-(trifluoromethyl)benzene.

[Formula 52]

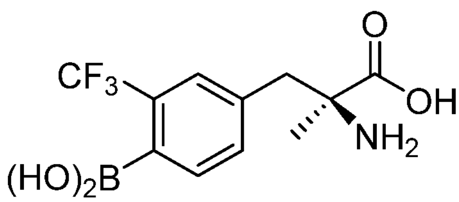

(S)-2-Amino-3-(4-borono-3-(trifluoromethyl)phenyl)-2-methylpropanoic acid

$^1H$ NMR (3.5% DCl in $D_2O$); 1.71 (s, 3H, α-Me), 3.24 (d, J = 14.5 Hz, 1H, β-H), 3.44 (d, J = 14.5 Hz, 1H, β-H), 7.51 (d, J = 8.0 Hz, 1H, ArH), 7.61-7.63 (m, 2H, ArH).

(Example 40)

The following compound was synthesized in the same manner as in Example 1 except that 4-bromo-1-(bromomethyl)-2-fluorobenzene was changed to 1-bromo-4-(bromomethyl)-2-(trifluoromethyl)benzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 53]

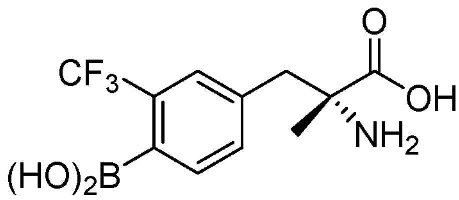

(R)-2-Amino-3-(4-borono-3-(trifluoromethyl)phenyl)-2-methylpropanoic acid

$^1H$ NMR (3.5% DCl in $D_2O$); 1.69 (s, 3H, α-Me), 3.23 (d, J = 14.5 Hz, 1H, β-H), 3.44 (d, J = 14.5 Hz, 1H, β-H), 7.50 (d, J = 8.0 Hz, 1H, ArH), 7.60-7.61 (m, 2H, ArH).

(Example 41)

The following compound was synthesized in the same manner as in Example 1 except that 4-bromo-1-(bromomethyl)-2-fluorobenzene was changed to 1-bromo-4-(bromomethyl)-2,5-difluorobenzene, and (R)-4,4-dibutyl-2,6-bis(3,4,5-trifluorophenyl)-4,5-dihydro-3H-dinaphtho[2,1-c:1',2'-e]azepinium bromide was changed to (S)-4,4-dibutyl-2,6-bis(3,4,5-trifluorophenyl)-4,5-dihydro-3H-dinaphtho[2,1-c:1',2'-e]azepinium bromide.

[Formula 54]

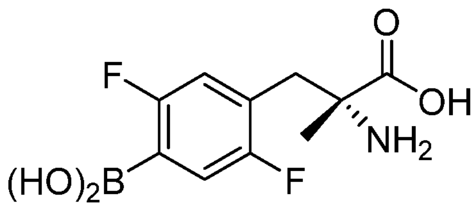

(R)-2-Amino-3-(4-borono-2,5-difluorophenyl)-2-methylp ropanoic acid

$^1H$ NMR (3.5% DCl in $D_2O$); 1.64 (s, 3H, α-Me), 3.36 (s, 2H, β-H), 7.31-7.33 (m, 2H, ArH).

(Example 42)

The following compound was synthesized in the same manner as in Example 1 except that tert-butyl 2-((4-chlorobenzylidene)amino)propanoate was changed to tert-butyl 2-((4-chlorobenzylidene)amino)butanoate.

[Formula 55]

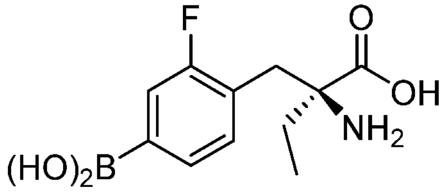

(S)-2-Amino-2-(4-borono-2-fluorobenzyl)-2-butanoic acid

$^1H$ NMR (3.5% DCl in $D_2O$); 1.02 (t, J = 7.5 Hz, 3H, $\alpha$-$CH_2CH_3$), 1.92-2.00 (m, 1H, $\alpha$-$CH_2CH_3$), 2.14-2.22 (m, 1H, $\alpha$-$CH_2CH_3$), 3.35 (s, 2H, $\beta$-H), 7.36 (d, J = 7.5 Hz, 1H, ArH), 7.47 (d, J = 11.0 Hz, 1H, ArH), 7.53 (dd, J = 1.0, 7.0 Hz, 1H, ArH).

(Example 43)

The following compound was synthesized in the same manner as in Example 1 except that 4-bromo-1-(bromomethyl)-2-fluorobenzene was changed to 1-bromo-4-(bromomethyl)-3-methylthiobenzene, and this was oxidized with m-chloroperbenzoic acid to sulfone before borylation.

[Formula 56]

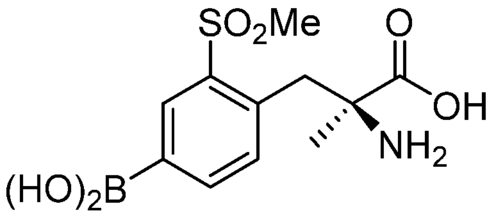

(S)-2-Amino-3-(4-borono-2-(methylsulfonyl)phenyl)-2-m ethylpropanoic acid

Retention time: 0.19 min
HRMS (ESI, $[M+H]^+$), calcd for 302.0870; found: 302.1029.

(Example 44)

The following compound was synthesized in the same manner as in Example 1 except that 4-bromo-1-(bromomethyl)-2-fluorobenzene was changed to 5-bromo-2-(bromomethyl)pyridine, and tert-butyl 2-((4-chlorobenzylidene)amino)propanoate was changed to N-(diphenylmethylene)glycine tert-butyl.

[Formula 57]

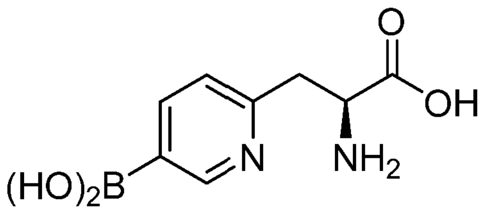

(S)-2-Amino-3-(5-boronopyridin-2-yl)propanoic acid

$^1H$ NMR ($D_2O$); 8.82 (brs, 1H, ArH), 8.73 (dd, J = 7.0, 9.5 Hz, 1H, ArH), 7.98 (d, J = 8.0 Hz, 1H, ArH), 4.49 (t, J = 7.5 Hz, 1H, CH), 3.65 (d, J = 7.5 Hz, 2H, $CH_2$).

(Example 45)

The following compound was synthesized in the same manner as in Example 1 except that 4-bromo-1-(bromomethyl)-2-fluorobenzene was changed to 5-bromo-2-(bromomethyl)pyridine.

[Formula 58]

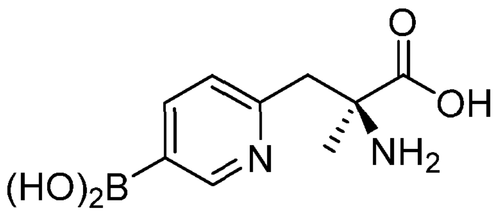

(S)-2-Amino-3-(5-boronopyridin-2-yl)-2-methylpropanoi c acid

$^1H$ NMR ($D_2O$); 8.87 (d, J = 3.5 Hz, 1H, ArH), 8.77 (dd, J = 2.5, 7.5 Hz, 1H, ArH), 7.96 (dd, J = 3.0, 7.5 Hz, 1H, ArH), 3.65 (d, J = 15.0 Hz, 1H, $CH_2$), 3.61 (d, J = 15.0 Hz, 1H, $CH_2$), 1.61 (s, 3H, $CH_3$).

(Example 46)

The following compound was synthesized in the same manner as in Example 1 except that 4-bromo-1-(bromomethyl)-2-fluorobenzene was changed to 5-bromo-2-(bromomethyl)pyrimidine.

[Formula 59]

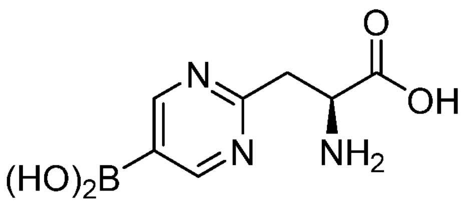

(S)-2-Amino-3-(5-boronopyrimidin-2-yl)propanoic acid Retention time: 0.19 min

HRMS (ESI, $[M+H]^+$), calcd for 212.0843; found: 212.0982.

(Example 47)

The following compound was synthesized in the same manner as in Example 1 except that 4-bromo-1-(bromomethyl)-2-fluorobenzene was changed to 4-bromo-2-(bromomethyl)thiophene.

[Formula 60]

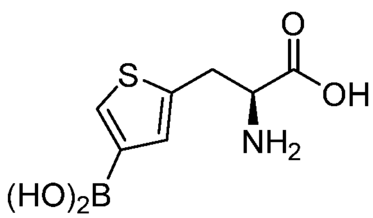

(S)-2-Amino-3-(4-boronothiophen-2-yl)propanoic acid Retention time: 0.26 min

HRMS (ESI, $[M+H]^+$), calcd for 216.0502; found: 216.0161

(Example 48)

Synthesis of (S)-2-amino-3-(5-borono-3-fluoropyridin-2-yl)propanoic acid

[Formula 61]

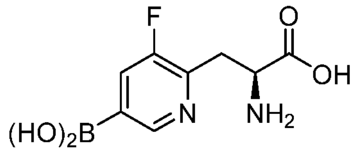

Step 1

Synthesis of methyl (S)-3-(5-bromo-3-fluoropyridin-2-yl)-2-((tert-butoxycarbony l)amino)propanoate

Under a nitrogen atmosphere, methyl (R)-2-((tert-butoxycarbonyl)amino)-3-iodopropanoate (2.98 g, 9.05 mmol) was added to the mixture of zinc powder (710 mg, 10.9 mmol), N,N-dimethylformamide (12 mL) and trimethylsilyl chloride (98.3 mg, 0.905 mmol) under ice cooling. When heat generation had subsided, 2,5-dibromo-3-fluoropyridine (3.00 g, 11.8 mmol) and bis(triphenylphosphine)palladium(II) dichloride (318 mg, 0.453 mmol) were added, and the mixture was stirred at 80°C for 1.5 hours. The reaction mixture was cooled to room temperature, filtered through Celite to remove a zinc residue, and the residue was washed with ethyl acetate (30 mL) . To the resulting filtrate was added a saturated aqueous ammonium chloride solution (10 mL), and the mixture was extracted with ethyl acetate (30 mL). The organic phase was washed with saturated saline (10 mL), dried over anhydrous sodium sulfate, and then concentrated under reduced pressure. The resulting residue was purified by silica gel column chromatography (hexane/ethyl acetate = 8/1), affording methyl (S)-3-(5-bromo-3-fluoropyridin-2-yl)-2-((tert-butoxycarbony l)amino)propanoate (1.71 g, 4.55 mmol, 50%) as a yellow oil.
Retention time: 2.52 min

HRMS (ESI, $[M+Na]^+$), calcd for 399.0332; found: 399.0714.

Step 2

Under a nitrogen atmosphere, bis(pinacolato)diboron (2.08 g, 8.19 mmol), $Pd(dba)_2$ (131 mg, 0.228 mmol), tricyclohexylphosphine (128 mg, 0.455 mmol), potassium acetate (1.34 g, 13.7 mmol), and 1,4-dioxane (30 mL) were added to methyl (S)-3-(5-bromo-3-fluoropyridin-2-yl)-2-((tert-butoxycarbony l)amino)propanoate (1.71 g, 4.55 mmol), and the mixture was stirred at 80°C for 6 hours. The reaction mixture was filtered through Celite to remove a palladium residue, and the residue was washed with ethyl acetate (30 mL) . Water (10 mL) was added to the resulting filtrate, and the mixture was extracted with ethyl acetate (30 mL). The organic phase was washed with saturated saline (10 mL), dried over anhydrous sodium sulfate, and then concentrated under reduced pressure. The resulting residue was passed through alumina (neutral) and distilled off with ethyl acetate for rough purification, affording a crude product (2.85 g) containing methyl

(S)-2-((tert-butoxycarbonyl)amino)-3-(3-fluoro-5-(4,4,5,5-t etramethyl-1,3,2-dioxaborolan-2-yl)pyridin-2-yl)propanoate as a yellow oil.

Sodium periodate (6.82 g, 31.9 mmol) and ammonium acetate (2.46 g, 31.9 mmol) were added to an acetone/water (2/1, 30 mL) solution of the crude product (2.85 g) obtained, and the mixture was stirred at room temperature for 15 hours. The insoluble matter was collected by filtration, and then acetone in the resulting filtrate was distilled off under reduced pressure. The resulting residue was extracted twice with ethyl acetate (50 mL). The organic phase was washed with saturated saline (10 mL), dried over anhydrous sodium sulfate, and then concentrated under reduced pressure. The resulting residue was powdered with diisopropyl ether (5 mL), affording a crude product containing

(S)-(6-(2-((tert-butoxycarbonyl)amino)-3-methoxy-3-oxopropy l)-5-fluoropyridin-3-yl) boronic acid (300 mg) as a light yellow powder.

Concentrated hydrochloric acid (4.4 mL) was added to the crude product (300 mg) obtained, and the mixture was stirred at 70°C for 22 hours. Water (20 mL) was added to the reaction mixture, and the aqueous phase was washed with dichloromethane (20 mL × 4). The aqueous phase was concentrated under reduced pressure, affording

(S)-2-amino-3-(5-borono-3-fluoropyridin-2-yl)propanoic acid hydrochloride (222 mg, 0.738 mmol, 16% for 3 steps) as a light yellow solid.

$^1H$ NMR ($D_2O$) ; 8. 67 (s, 1H, ArH), 8.33 (d, J = 8.5 Hz, 1H, ArH), 4.39 (dd, J = 5.5, 8.0 Hz, 1H, CH), 3.65 (dd, J = 5.5, 15.0 Hz, 1H, $CH_2$), 3.59 (dd, J = 8.0, 15.0 Hz, 1H, $CH_2$).

(Example 49)

The following compound was synthesized in the same manner as in Example 48 except that 2,5-dibromo-3-fluoropyridine was changed to 5-bromo-2-iodo-3-methylpyridine, and $Pd(PPh_3)_2Cl_2$ was changed to $Pd_2(dba)_3$ and tri(ortho-tolyl)phosphine.

[Formula 62]

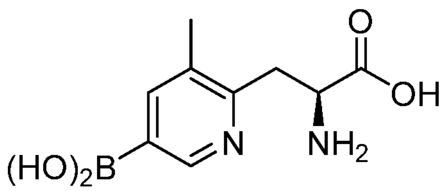

(S)-2-Amino-3-(5-borono-3-methylpyridin-2-yl)propanoi c acid

$^1H$ NMR ($D_2O$); 8.68 (s, 1H, ArH), 8.61 (s, 1H, ArH), 4.36 (dd, J = 5. 0, 10.0 Hz, 1H, CH), 3.66 (dd, J = 5. 0, 14.5 Hz, 1H, $CH_2$), 3.60 (dd, J = 10.0, 14.5 Hz, 1H, $CH_2$), 2.54 (s, 3H, $CH_3$).

(Example 50)

The following compound was synthesized in the same manner as in Example 48 except that 2,5-dibromo-3-fluoropyridine was changed to 2,5-dibromo-3-chloropyridine.

[Formula 63]

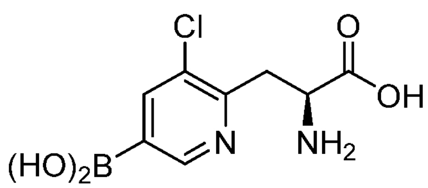

(S)-2-Amino-3-(5-borono-3-chloropyridin-2-yl)propanoi c acid

$^1H$ NMR ($D_2O$); 8.72 (d, J = 1.5 Hz, 1H, ArH), 8.53 (d, J = 1.5 Hz, 1H, ArH), 4.42 (dd, J = 6.0, 8.5 Hz, 1H, CH), 3.71 (dd, J = 6.0, 15.0 Hz, 1H, $CH_2$), 3.63 (dd, J = 8.5, 15.0 Hz, 1H, $CH_2$).

(Example 51)

The following compound was synthesized in the same manner as in Example 48 except that 2,5-dibromo-3-fluoropyridine was changed to 5-bromo-2-iodo-4-methylpyridine.

[Formula 64]

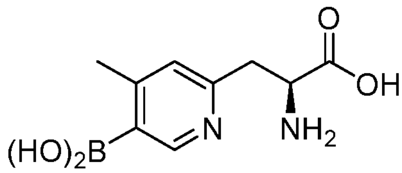

(S)-2-Amino-3-(5-borono-4-methylpyridin-2-yl)propanoi

c acid

$^1H$ NMR ($D_2O$); 8.56 (s, 1H, ArH), 7.77 (s, 1H, ArH), 4.43 (t, J = 7.5 Hz, 1H, CH), 3.55 (d, J = 7.5 Hz, 2H, $CH_2$), 2.64 (s, 3H, $CH_3$).

(Example 52)

Production of 2-amino-5-borono-2,3-dihydro-1H-indene-2-carboxylic acid

[Formula 65]

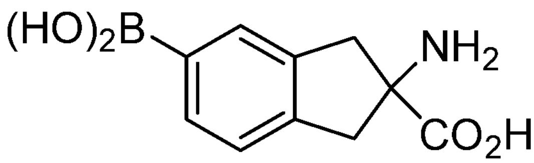

(Step 1)

[Formula 66]

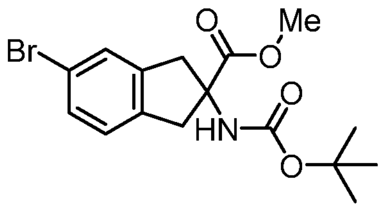

Production of methyl 2-amino-5-bromo-2,3-dihydro-1H-indene-2-carboxylate

A mixed liquid of 2-amino-5-bromo-2,3-dihydro-1H-indene-2-carboxylic acid hydrochloride (0.5 g, 1.7 mmol), methanol (5 mL), and concentrated sulfuric acid (0.25 mL) was heated to reflux for 16 hours. The reaction mixture was added to saturated aqueous sodium bicarbonate solution (100 mL), and the resulting mixture was extracted with dichloromethane (50 mL × 2), and then the organic phase was washed with semi-saturated saline (100 mL) . The organic phase was dried over anhydrous sodium sulfate and then concentrated under reduced pressure, affording methyl 2-amino-5-bromo-2,3-dihydro-1H-indene-2-carboxylate (0.43 g, yield 93.6%) as a light brown oil.

To a mixture of methyl 2-amino-5-bromo-2,3-dihydro-1H-indene-2-carboxylate (0.43 g, 1.6 mmol), dichloromethane (15 mL), and triethylamine (0.45 mL) was added dropwise a solution of di-tert-butyl dicarbonate (0.55 mL) in dichloromethane (3 mL) over 2 minutes under ice cooling. The reaction mixture was stirred at room temperature overnight, then added to dichloromethane (100 mL), and washed with semi-saturated saline (100 mL × 2) . The organic phase was dried over anhydrous sodium sulfate and then concentrated under reduced pressure, and the resulting residue

was purified by silica gel column chromatography (hexane/ethyl acetate), affording methyl 5-bromo-2-(tert-butoxycarbonyl)amino-2,3-dihydro-1H-indene-2-carboxylate (0.26 g, yield 43.9%) as a white solid.

$^1$H NMR ($CDCl_3$); 1.42 (s, 9H), 3.04-3.51 (m, 4H), 3.76 (s, 3H), 5.07 (s, 1H), 6.97-7.33 (m, 4H).

(Step 2)

[Formula 67]

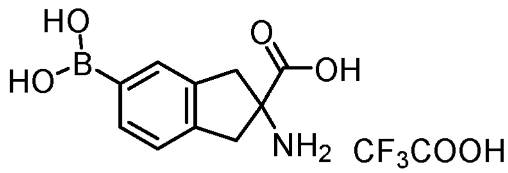

A mixed liquid of methyl 5-bromo-2-(tert-butoxycarbonyl)amino-2,3-dihydro-1H-indene-2-carboxylate (0.24 g, 0.65 mmol), bis(pinacolato) diboron (0.2 g), [1,1'-bis(diphenylphosphino)ferrocene]dichloropalladium(II) (0.03 g), potassium acetate (0.1 g), and dimethyl sulfoxide (3 mL) was purged with nitrogen, and then stirred at 95°C for 8 hours. Water (100 mL) was added to the reaction mixture, then the mixture was extracted with ethyl acetate (100 mL). The organic phase was washed with saturated saline (100 mL), then dried over anhydrous sodium sulfate, and concentrated under reduced pressure. The resulting residue was purified by silica gel column chromatography (hexane/ethyl acetate), affording methyl 2-(tert-butoxycarbonyl)amino-5-(4,4,5,5-tetramethyl-1,3,2-d ioxaboran-2-yl)-2,3-dihydro-1H-indene-2-carboxylate (0.25 g, yield 92.2%) as a colorless amorphous.

Sodium periodate (0.19 g) and ammonium acetate (0.07 g) were added to the mixture of methyl 2-(tert-butoxycarbonyl)amino-5-(4,4,5,5-tetramethyl-1,3,2-d ioxaborolan-2-yl)-2,3-dihydro-1H-indene-2-carboxylate (0.09 g, 0.22 mmol), acetone (7 mL), and water (3.5 mL), and the mixture was stirred at room temperature overnight. The reaction mixture was added to ethyl acetate (100 mL), and washed with semi-saturated saline (150 mL × 2). The organic phase was dried over anhydrous sodium sulfate and then concentrated under reduced pressure, affording methyl 2-(tert-butoxycarbonyl)amino-5-borono-2,3-dihydro-1H-indene -2-carboxylate (0.08 g, yield 99%) as a white amorphous.

To a solution of methyl 5-borono-2-(tert-butoxycarbonyl)amino-2,3-dihydro-1H-indene -2-carboxylate (0.17 g, 0.5 mmol) in methanol (2 mL) was added a 1 M aqueous sodium hydroxide solution (2 mL) under ice cooling. The mixture was stirred at room temperature for 3 hours. The reaction mixture was adjusted to about 3 with 1 M hydrochloric acid under ice cooling, and then the reaction mixture was extracted with ethyl acetate (100 mL) . The organic phase was washed with saturated saline (100 mL), then dried over anhydrous sodium sulfate, and then concentrated under reduced pressure, affording 5-borono-2-(tert-butoxycarbonyl)amino-2,3-dihydro-1H-indene -2-carboxylic acid (0.15 g, yield 93. 4%) as a white amorphous.

Trifluoroacetic acid (1.44 mL) was added to 5-borono-2-(tert-butoxycarbonyl)amino-2,3-dihydro-1H-indene -2-carboxylic acid (0.15 g, 0.47 mmol), and the mixture was stirred at room temperature for 2 hours. The reaction mixture was concentrated under reduced pressure, then toluene (3 mL) was added thereto, and the resulting mixture was further concentrated under reduced pressure. The resulting residue was powdered with dichloromethane, affording trifluoroacetate of 2-amino-5-borono-2,3-dihydro-1H-indene-2-carboxylic acid (0.13 g, yield 82.6%) as a white powder.

$^1$H NMR ($D_2O$); 3.39 (d, J = 18 Hz, 2H, $CH_2$), 3.79 (d, J = 18 Hz, 2H, $CH_2$), 7.38 (d, J = 7.5 Hz, 1H, ArH), 7.67-7.68 (m, 2H, ArH).

(Example 53)

The following compound was synthesized in the same manner as in Example 52 except that 2-amino-5-bromo-2,3-dihydro-1H-indene-2-carboxylic acid hydrochloride was changed to 2-amino-7-bromo-1,2,3,4-tetrahydronaphthalene-2-carboxylic acid.

[Formula 68]

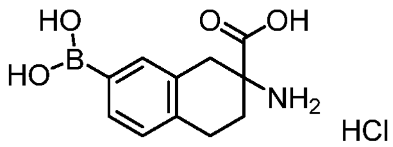

2-Amino-7-borono-1,2,3,4-tetrahydronaphthalene-2-carb oxylic acid hydrochloride

$^1$H NMR ($D_2O$); 2.17-2.20 (m, 1H, $CH_2$), 2.38-2.42 (m, 1H, $CH_2$), 2.90-3.11 (m, 3H, $CH_2$), 3.47-3.50 (m, 1H, $CH_2$), 7.20-7.29 (m, 2H, ArH), 7.54-7.60 (m, 1H, ArH).

[Uptake test]

1. Construction of a system for evaluation of LAT1 and LAT2-selective uptake

(1) Preparation of human LAT1 and LAT2 highly expressing HEK 293 cell lines

HEK 293 cells that stably highly express human LAT1 and LAT2 respectively were prepared according to the method described in the article of Khunweeraphong, N et al. (Journal of Pharmacology Science, 2012, vol. 119, pp 368-380) . A shuttle vector DNA (LAT1: EX-H 4509-M02, LAT2: EX-U0514-M02, manufactured by GeneCopoeia) having ampicillin and neomycin resistance markers and having full-length cDNA of human LAT1 or LAT2 inserted under the promoter of cytomegalovirus (CMV) was constructed. This vector was transfected into HEK 293 cells by a method using Lipofectamine (registered trademark) 2000 (Invitrogen) in accordance with the manufacturer's instructions. Thereafter, the stably expressing cell clone of the transgene was selected by the limiting dilution method in the presence of 0.9 mg/mL Geneticin (registered trademark), and a cell clone in which the uptake of L-boronophenylalanine (L-BPA) was enhanced by about 2 to about 5 times as compared with HEK 293 cells before transfection was obtained. This cell was passaged and used for evaluation of LAT1 and LAT2-selective uptake.

(2) Evaluation of LAT1 and LAT2-selective uptake

As a method for evaluating cell uptake, the same method as in the article of Khunweeraphong, N et al. (Journal of Pharmacology Science, 2012, vol. 119, pp 368-380) was used. Each of the cells obtained in (1) was used for evaluation. Radioactive isotopes were not used, a substrate concentration was 0.1 mM, and 2 mM BCH (2-amino-2-norbonanecarboxylic acid) was used as an LAT1 and LAT2 inhibitor. The reacted cells were collected with 0.05% Tween 20 and the concentration of the 4-borono-phenylalanine derivative in the cell fluid was determined. Quantification of the 4-boronophenylalanine derivative in cells was performed in accordance with the method described in the article of Hattori, Y, et al. (Sensors 2017, 17, 2436), using 2-(2-hydroxyphenyl)pyridine (boron sensor 5) as a boron sensor. In the evaluation of cell uptake, in consideration of variations between experiments, the cell uptake was evaluated as a value (LAT1 selectivity) relative to the uptake of L-BPA as a comparative object performed on the same day.

LAT1 selectivity = (quantitative value of uptake of the compound in LAT1 cells/quantitative value of uptake of control L-BPA in LAT1 cells)/(quantitative value of uptake of the compound in LAT2 cells/quantitative value of uptake of control L-BPA in LAT2 cells)

That is, it is as follows.

[Mathematical Formula 1]

LAT1 selectivity = (quantitative value of uptake of the compound in LAT1 cells/quantitative value of uptake of control L-BPA in LAT1 cells)/(quantitative value of uptake of the compound in LAT2 cells/quantitative value of uptake of control L-BPA in LAT2 cells)

As a result of the uptake evaluation performed by comparing the compounds of Examples with L-BPA, a plurality of compounds showing high values of LAT1 selectivity were found. Among them, in particular, the following compounds showed extremely high values, namely twice or more:

(S)-2-amino-3-(4-borono-2-fluorophenyl)-2-methylpropa noic acid;
(R)-2-amino-3-(4-borono-2-fluorophenyl)-2-methylpropa noic acid;
(S)-2-amino-3-(4-borono-2-methylphenyl)-2-methylpropa noic acid;
(R)-2-Amino-3-(4-borono-2-methylphenyl)-2-methylpropa noic acid
(S)-2-amino-3-(4-borono-2-(trifluoromethyl)phenyl)-2-methylpropanoic acid;
(R)-2-amino-3-(4-borono-2-(trifluoromethyl)phenyl)-2-methylpropanoic acid;
(S)-2-amino-3-(4-borono-2-methoxyphenyl)-2-methylprop anoic acid;
(S)-2-amino-3-(4-borono-2-chlorophenyl)-2-methylpropa noic acid;
(S)-2-amino-3-(4-borono-2,6-difluorophenyl)-2-methylp ropanoic acid;
(S)-2-amino-3-(4-borono-5-chloro-2-fluorophenyl)-2-me thylpropanoic acid;
(S)-2-amino-3-(4-borono-2,3-difluorophenyl)-2-methylp ropanoic acid;
(S)-2-amino-3-(4-borono-3-fluorophenyl)-2-methylpropa noic acid;
(R)-2-amino-3-(4-borono-3-fluorophenyl)-2-methylpropa noic acid;
(S)-2-amino-3-(4-borono-2-chloro-5-fluorophenyl)-2-me thylpropanoic acid;
(S)-2-amino-3-(4-borono-3-methylphenyl)-2-methylpropa noic acid;
(S)-2-amino-3-(4-borono-2-(methylthio)phenyl)-2-methy lpropanoic acid;
(S)-2-amino-3-(4-borono-2-(difluoromethyl)phenyl)-2-m ethylpropanoic acid;
(R)-2-amino-3-(4-borono-2-(difluoromethyl)phenyl)-2-m ethylpropanoic acid;
(S)-2-amino-3-(4-borono-3-chlorophenyl)-2-methylpropa noic acid;
(S)-2-amino-3-(5-boronopyridin-2-yl)propanoic acid;
(S)-2-amino-3-(5-borono-3-fluoropyridin-2-yl)propanoi c acid;
(S)-2-amino-3-(5-borono-3-methylpyridin-2-yl)propanoi c acid;
(S)-2-amino-3-(5-borono-3-chloropyridin-2-yl)propanoi c acid; and
(S)-2-amino-3-(5-borono-4-methylpyridin-2-yl)propanoi c acid.

[Uptake test 2]

Test for evaluating uptake into tumor cells

On a 100 mm dish are seeded $1.5 \times 10^6$ human tongue cancer cells (SAS cells), human glioma cells (A172 cells), or human breast cancer cells (MCF-7), and pre-culture is performed at 37°C in an atmosphere of 5% $CO_2$ for 24 hours. The culture fluid is removed by suction, a culture fluid containing 1 mM of the agent of each Example or Reference Example is added, and then exposed and cultured at 37°C in an atmosphere of 5% $CO_2$ for 3 hours. After the culture fluid is removed by suction, the cells are washed once using PBS, and then trypsinized to recover the cells. The number of the cells recovered is counted and packed by centrifugation, then $HClO_4$ (60%, 0.3 ml) and $H_2O_2$ (31%, 0.6 mL) are added, the mixture is heated at 75°C overnight, and an ashed solution is thereby prepared. The ashed solution is filled up to 5 mL with pure water, and then filtered using a 5C filter paper, the boron concentration in the solution is measured using 710 ICP-OES manufactured by Agilent Technologies Inc., and the amount ($\mu$g) of boron per $10^7$ cells is thereby determined. In the evaluation of cell uptake, in consideration of variations between experiments, the cell uptake is evaluated as a value relative to the uptake of L-BPA of Reference Example as a comparative object performed on the

same day.

[Single dose toxicity test]

In a beaker, 17.6 mL of 1 mol/L NaOH was put, and 24 mL of water for injection was further added and mixed. To this was added 2.52 g of D-sorbitol. After confirming complete dissolution, 17.1 mL of 1 mol/L hydrochloric acid was added to adjust the pH to 7.4 to 7.8. The mixture was transferred to a measuring cylinder, the beaker was washed with water for injection, and the washing liquid was transferred to the measuring cylinder. This operation was repeated as much as possible, and the volume of the liquid in the measuring cylinder was increased to 80 mL while the beaker was washed. The mixture was filtration-sterilized with a filter (Millex GV, pore size: 0.22 μm, Merck, sterilized disposable product), affording a 3.15% D-sorbitol solution.

On the other hand, 10.1 mL of a 1 mol/L aqueous sodium hydroxide solution was put in a beaker, and 13.8 mL of water for injection was further added and mixed. To this were added 2.30 g of test substance and 1.45 g of D-sorbitol. After confirming complete dissolution, 1.36 mL of 1 mol/L hydrochloric acid was added to adjust the pH to 7.4 to 7.8. The mixture was transferred to a measuring cylinder, the beaker was washed with water for injection, and the washing liquid was transferred to the measuring cylinder. This operation was repeated as much as possible, and the volume of the liquid in the measuring cylinder was increased to 46 mL while washing the beaker. The mixture was filtration-sterilized with a filter (Millex GV, pore size: 0.22 μm, Merck, sterilized disposable product), affording a liquid with a concentration of 50 mg/mL.

An animal was placed in a fixation device (Bollman cage) and injected into the tail vein at a rate of 1 mL/kg/min with a microprocessor single syringe pump (Pump 11 Elite, Harvard Apparatus Inc.) using a 10 mL polypropylene syringe filled with the administration liquid and a 24 gauge indwelling needle (Surflow F&F, Terumo Corporation, all sterilized disposable products) . At doses of 250, 500, and 1000 mg/kg, 5 animals in each group were administered, and after completion of the administration, the animals were returned to the breeding cages.

The observation period was 8 days including the administration day. The administration day was defined as Day 1, the day following the administration day was defined as Day 2, and the subsequent days are indicated based on those definitions. The observation frequency was set to 6 times (immediately before administration, 5 minutes, 30 minutes, 60 minutes, 2 hours, and 4 hours after the end of administration) on the administration day, and once a day on and after the day following the administration day. Individual observation was performed from the outside of the cages, and animals suspected of having abnormality were taken out from the cages and observed. The time of body weight measurement was set to Day 1 (before administration), Day 4, and Day 8.

As a result of this test, with the compound (S)-2-amino-3-(4-borono-2-fluorophenyl)-2-methylpropanoic acid and the compound (S)-2-amino-3-(4-borono-2-chlorophenyl)-2-methylpropanoic acid, no serious change due to the administration of the test substance was observed in any of the administration groups throughout the entire observation period.

[Distribution test using tumor-bearing mouse]

Human pancreatic cancer cells T3M-4 cells are cultured using a HAM-F12 medium. Cells are detached with trypsin solution and collected by centrifugation. Cells are suspended in PBS (-) to $4 \times 10^6$ cells/100 μL. The cell suspension is injected (100 μL/mouse) subcutaneously into the right lower limb of BALB/c nu/nu mice (male, 4 weeks old) using a 26 G needle. Thereafter, the size of the tumor formed is visually examined for 3 to 4 weeks, and when the tumor size reaches about 4 mm to about 10 mm, the sample is used for distribution experiment. The administration agent is correspondingly prepared with an agent, for example, by adding fructose or sorbitol, and physiological saline or PBS (-) is used as the diluent. To tumor-bearing mice, 100 μL of the agent with a sample concentration of 10 mg/mL as a standard is administered at their tail vein. The mice are sacrificed over time and dissected, and organ weights are measured. The extracted organ is ashed with nitric acid, and the amount of boron is quantified by ICP-MS or ICP-OES.

[Measurement of Km value]

As a method for evaluating Km, the cell uptake method described in the article of Khunweeraphong, N, et al. (Journal of Pharmacology Science, 2012, vol. 119, pp 368-380) is used, and the uptake time is 2 to 3 minutes. At this time, radioactive isotopes are not used, and cells reacted at a plurality of substrate concentrations are recovered with 0.05% Tween 20, and the concentration of the boron compound in the obtained cell fluid is determined. As the cells, HEK 293 cells that stably express Human LAT1 transporter or Human LAT2 transporter established according to (Journal of Pharmacology Science, 2012, vol. 119, pp 368-380) are used.

Quantification of the boron compound in cells is performed in accordance with the method described in the article of Hattori, Y, et al. (Sensors 2017, 17, 2436), using 2-(2-hydroxyphenyl)pyridine (boron sensor 5) as a boron

sensor. In the evaluation, Km is calculated using Lineweaver-Burk plot.

[Measurement of efflux]

After making LAT1-enhanced HEK 293 cells uptake the substrate, an effluent is added and the boron compound remaining in the cells is measured. HBSS (Na+-Free) and 0.05 mM leucine are used as the LAT1 effluent at a substrate concentration of 0.1 mM, and the concentration of the boron compound in the cell fluid obtained by collecting the reacted cells with 0.05% Tween 20 is determined. The efflux time is set to 1 to 10 minutes.

Quantification of the boron compound in cells is performed in accordance with the method described in the article of Hattori, Y, et al. (Sensors 2017, 17, 2436), using 2-(2-hydroxyphenyl)pyridine (boron sensor 5) as a boron sensor.

In the evaluation of cell uptake, the cell uptake is evaluated as a value relative to the cell fluid before efflux as a comparative object.

[Evaluation of solubility in water]

An appropriate amount of the compound of each Example is weighed and dissolved in water. Hydrochloric acid or an aqueous sodium hydroxide solution is added dropwise, and the mixture is made neutral while checking with a pH test paper. The agent prepared is analyzed by ICP to calculate the boron concentration, which is taken as the sample concentration.

[Metabolic stability test]

Using commercially available pooled human liver microsomes, a target compound is reacted for a prescribed period of time, and the reacted sample is compared to an unreacted sample to calculate a residual ratio. Then, the degree of hepatic metabolism is evaluated.

A reaction (oxidative reaction) is performed at 37°C for 0 minutes or 30 minutes in 0.2 mL of a buffer (50 mmol/L tris-HCl pH 7.4, 150 mmol/L potassium chloride, 10 mmol/L magnesium chloride) containing 0.5 mg protein/mL of human liver microsome in the presence of 1 mmol/L NADPH. After the reaction, 50 μL of the reaction liquid is added to 100 μL of a methanol/acetonitrile = 1/1 (v/v) solution, mixed, and centrifuged at 3000 rpm for 15 minutes. The test compound in the centrifugal supernatant is quantified by LC/MS-MS, and the residual amount of the test compound after the reaction is calculated with the amount of the compound at 0 minute reaction defined as 100%.

[Metabolic stability test]

Using various commercially available frozen hepatocytes, a target compound is reacted for a prescribed period of time, and the reacted sample is compared to an unreacted sample to calculate a residual ratio. Then, the degree of hepatic metabolism is evaluated.

A reaction is carried out at 37°C for 0, 1, or 2 hours in William E culture medium containing $1.0 \times 10^6$ cells/mL of frozen hepatocytes. After the reaction, 120 μL of a methanol/acetonitrile = 1/1 (v/v) solution is added to 30 μL of the reaction liquid, mixed, and centrifuged at 3000 rpm for 15 minutes. The test compound in the centrifugal supernatant is quantified by LC/MS-MS, and the residual amount of the test compound after the reaction is calculated with the amount of the compound at 0 minute reaction defined as 100%.

[Protein binding test]

The serum protein unbinding rate of the compound of the invention is measured using various serums.

The reaction conditions are as follows. Evaluation method: equilibrium dialysis; reaction time: 24 h; reaction temperature: 37°C; concentration of the compound of the invention: 2 μg/mL

A serum sample having the above-mentioned compound concentration is prepared by adding a test liquid to a serum and stirring the mixture. The serum sample is added to one equilibrium dialysis cell and a phosphate buffered saline (PBS) is added to another equilibrium dialysis cell, and then equilibrium dialysis is performed at 37°C for 24 hours. The amount of the compound in the sample collected from each cell is measured by LC/MS-MS.

[Examples of pharmaceutical preparation] Injection

The compound of the present invention is dissolved in water or a buffer solution to prepare an injection. In the case of being insoluble in water, an aqueous solution of sodium hydroxide is added and the compound is dissolved, and the mixture is neutralized with hydrochloric acid to prepare an injection. From the osmotic relationship, physiological

saline, phosphate buffer, monosaccharides, disaccharides, or other carbohydrates may also be added.

## Claims

1. A compound represented by the following formula (I) or a pharmaceutically acceptable salt thereof:

[Formula 69]

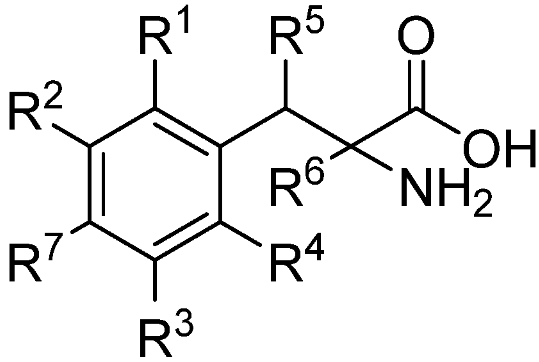

(I)

in the formula (I),

$R^1$, $R^2$, $R^3$, and $R^4$ each independently represent H, halogen, hydroxy, cyano, C1-6 alkyl, C1-6 alkoxy, benzyloxy, C1-6 alkoxy C1-6 alkyl, nitro, C1-6 haloalkyl, aminocarbonyl, C1-C6 alkylaminocarbonyl ($CONR^8R^9$ ($R^8$ and $R^9$ each independently represent H or C1-6 alkyl)), C1-C6 alkoxycarbonyl, C1-C6 alkylcarbonyl, $COOR^{10}$ ($R^{10}$ represents H or C1-6 alkyl, amino, alkylamino ($NR^{11}R^{12}$ ($R^{11}$ and $R^{12}$ each independently represent H or C1-6 alkyl)), haloalkylsulfanyl, haloalkylsulfinyl, haloalkylsulfonyl, C1-6 alkylthio, C1-6 alkylsulfinyl, C1-6 alkylsulfonyl, aminosulfonyl, sulfo, or sulfamoyl;
$R^5$ represents H, hydroxy, C1-6 alkyl, or halogen;
$R^6$ represents C1-6 alkyl;
$R^7$ represents boronic acid ($-B(OH)_2$), a boronic acid ester, or a boronic acid amide,
with the proviso that when $R^1$, $R^2$, $R^3$, and $R^4$ are all H, $R^5$ represents hydroxy or F, or when $R^1$, $R^2$, $R^3$, and $R^4$ are all H, $R^6$ represents C2-6 alkyl.

2. The compound or a pharmaceutically acceptable salt thereof according to claim 1, wherein $R^6$ represents methyl or ethyl.

3. The compound or a pharmaceutically acceptable salt thereof according to claims 1 or 2, wherein $R^7$ represents boronic acid ($B(OH)_2$) or a pinacol ester of boronic acid.

4. The compound or a pharmaceutically acceptable salt thereof according to any one of claims 1 to 3, wherein $R^5$ represents H.

5. The compound or a pharmaceutically acceptable salt thereof according to any one of claims 1 to 4, wherein any one or two or more of $R^1$, $R^2$, $R^3$, and $R^4$ are each independently Cl, F, C1-4 alkyl, C1-4 alkoxy, C1-4 alkylthio, $CH_2X$, $CHX_2$, or $CX_3$ (X represents F) .

6. An L-type amino acid transporter 1 (LAT1) -selective agent including the compound or a pharmaceutically acceptable salt thereof according to any one of claims 1 to 5.

7. An agent for BNCT, including the compound or a pharmaceutically acceptable salt thereof according to any one of claims 1 to 5.

**8.** A diagnostic agent containing a radioisotope, the diagnostic agent including the compound or a pharmaceutically acceptable salt thereof according to any one of claims 1 to 5.

**9.** A compound represented by the following formula (III) or a pharmaceutically acceptable salt thereof:

[Formula 70]

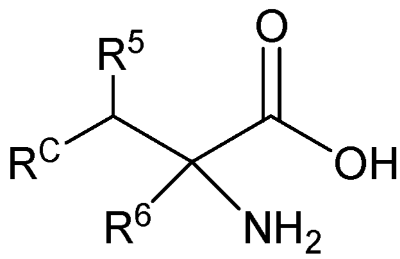

(III)

in the formula (III),

$R^c$ represents a heterocyclic ring having one group substituted with boronic acid ($-B(OH)_2$), a boronic acid ester, or a boronic acid amide and optionally substituted at 1 to 3 positions with halogen, hydroxy, cyano, C1-6 alkyl, C1-6 alkoxy, benzyloxy, C1-6 alkoxy C1-6 alkyl, nitro, C1-6 haloalkyl, aminocarbonyl, C1-C6 alkylaminocarbonyl ($CONR^8R^9$ ($R^8$ and $R^9$ each independently represent H or C1-6 alkyl)), C1-C6 alkoxycarbonyl, C1-C6 alkylcarbonyl, $COOR^{10}$ ($R^{10}$ represents H or C1-6 alkyl, amino, alkylamino ($NR^{11}R^{12}$ ($R^{11}$ and $R^{12}$ each independently represent H or C1-6 alkyl)), haloalkylsulfanyl, haloalkylsulfinyl, haloalkylsulfonyl, C1-6 alkylthio, C1-6 alkylsulfinyl, C1-6 alkylsulfonyl, aminosulfonyl, sulfo, or sulfamoyl;
the heterocyclic ring is one selected from the group consisting of pyridine, pyrimidine, thiophene, triazine, pyrrole, pyrazine, oxazole, isoxazole, oxadiazole, thiadiazole, isothiazole, and thiazole,
$R^5$ represents H, hydroxy, C1-6 alkyl, or halogen; and $R^6$ represents C1-6 alkyl.

**10.** A compound represented by the following formula (IV) or a pharmaceutically acceptable salt thereof:

[Formula 71]

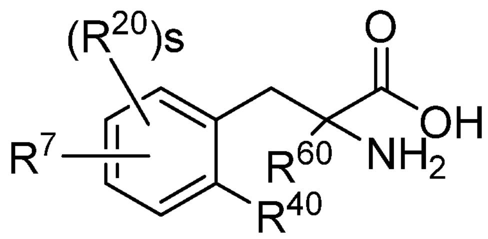

(IV)

in the formula (IV),

$R^{20}$ independently at each occurrence represents H, halogen, hydroxy, cyano, C1-6 alkyl, C1-6 alkoxy, benzyloxy, C1-6 alkoxy C1-6 alkyl, nitro, C1-6 haloalkyl, aminocarbonyl, C1-C6 alkylaminocarbonyl ($CONR^8R^9$ ($R^8$ and $R^9$ independently represent H or C1-6 alkyl)), C1-C6 alkoxycarbonyl, C1-C6 alkylcarbonyl, $COOR^{10}$ ($R^{10}$ represents H or C1-6 alkyl, amino, alkylamino ($NR^{11}R^{12}$ ($R^{11}$ and $R^{12}$ each independently represent H or C1-6 alkyl)), haloalkylsulfanyl, haloalkylsulfinyl, haloalkylsulfonyl, C1-6 alkylthio, C1-6 alkylsulfinyl, C1-6 alkylsulfonyl, aminosulfonyl, sulfo, or sulfamoyl;
$R^7$ represents boronic acid ($-B(OH)_2$), a boronic acid ester, or a boronic acid amide;
$R^{40}$ and $R^{60}$ together form a ring structure fused to a benzene ring, wherein the ring structure represents

unsubstituted C5-7 cycloalkyl or unsubstituted C6-7 cycloalkenyl;
s represents any integer of 1 to 3,
with the proviso that

[Formula 72]

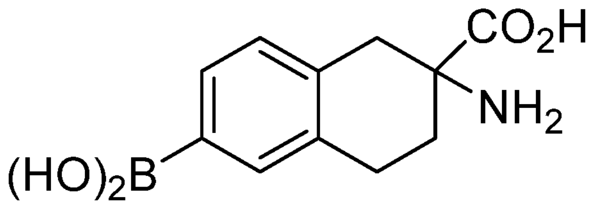

is excluded.

**INTERNATIONAL SEARCH REPORT**

International application No. **PCT/JP2022/018258**

**A. CLASSIFICATION OF SUBJECT MATTER**

***C07F 5/02***(2006.01)i; ***A61K 31/69***(2006.01)i; ***A61K 41/00***(2020.01)i; ***A61K 51/00***(2006.01)i; ***A61K 51/04***(2006.01)i; ***A61P 35/00***(2006.01)i
FI: C07F5/02 C CSP; A61P35/00; A61K41/00; A61K31/69; A61K51/00 200; A61K51/04 200

According to International Patent Classification (IPC) or to both national classification and IPC

**B. FIELDS SEARCHED**

Minimum documentation searched (classification system followed by classification symbols)

C07F5/02; A61K31/69; A61K41/00; A61K51/00; A61K51/04; A61P35/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y<br>A | JP 2018-024668 A (STELLA PHARMA CO., LTD.) 15 February 2018 (2018-02-15)<br>claims, paragraph [0003] | 1-8<br>9-10 |
| Y<br>A | PL 227525 B1 (UNIWERSYTET MIKOLAJA KOPERNIKA W TORUNIU) 29 December 2017 (2017-12-29)<br>claim 1 | 1-8<br>9-10 |
| Y<br>A | HARADA, S. et al., Catalytic asymmetric synthesis of α-methyl-p-boronophenylalanine, Bioorganic & Medicinal Chemistry Letters, 2018, 28, 1915-1918<br>p. 1917, compound (S)-11 | 1-8<br>9-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 June 2022** | **05 July 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/018258**

**C. DOCUMENTS CONSIDERED TO BE RELEVANT**

| Category* | Citation of document, with indication, where appropriate, of the relevant passages | Relevant to claim No. |
|---|---|---|
| Y | ZAIDLEWICZ, M. et al., Synthesis of boronated phenylalanine analogues with a quaternary center for boron neutron capture therapy, ARKIVOC, 2004, (iii), 11-21<br>p. 12, compound 3 | 1-8 |
| A | | 9-10 |
| X | PL 215215 B1 (UNIWERSYTET MIKOLAJA KOPERNIKA) 29 November 2013 (2013-11-29)<br>claims | 10 |
| A | | 1-9 |

Form PCT/ISA/210 (second sheet) (January 2015)

**INTERNATIONAL SEARCH REPORT**
**Information on patent family members**

International application No.
**PCT/JP2022/018258**

| Patent document cited in search report | Publication date (day/month/year) | Patent family member(s) | Publication date (day/month/year) |
|---|---|---|---|
| JP 2018-024668 A | 15 February 2018 | US 2016/0311836 A1<br>claims, paragraph [0003]<br>EP 3085687 A1 | |
| PL 227525 B1 | 29 December 2017 | (Family: none) | |
| PL 215215 B1 | 29 November 2013 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008214319 A


### Non-patent literature cited in the description


- **KHUNWEERAPHONG, N et al.** *Journal of Pharmacology Science,* 2012, vol. 119, 368-380
- **HATTORI, Y et al.** *Sensors,* 2017, vol. 17, 2436
- *Journal of Pharmacology Science,* 2012, vol. 119, 368-380